# EUROPEAN PATENT APPLICATION

(11) **EP 4 537 873 A1**
(43) Date of publication of application: **16.04.2025**
(21) Application number: 23203607.9
(22) Date of filing: 13.10.2023
(51) Int. Cl.: A61M 5/20, A61M 5/315

(54) **ELECTRONIC MODULE AND DRUG DELIVERY DEVICE**

(71) Applicant: medmix Switzerland AG, 9469 Haag (Rheintal) (CH)
(72) Inventor: CATTERMOLE, David, 70567 Stuttgart (DE); O'HARE, Aidan, 70567 Stuttgart (DE); JONES, Matthew, 70567 Stuttgart (DE); MARSH, William, 70567 Stuttgart (DE); BECHTOLD, Herbert, 70567 Stuttgart (DE); GAUL, Stefan, 70567 Stuttgart (DE); LURF, Robert, 70567 Stuttgart (DE)
(74) Representative: Manitz Finsterwald Patent- und Rechtsanwaltspartnerschaft mbB

(57) **Abstract**

An electronic module for a drug delivery device comprises a distance sensor configured to generate a signal indicating a distance between the distance sensor and at least one component of the drug delivery device connected to the electronic module, and a processing unit configured to receive the generated signal from the distance sensor and to acquire, based on the received signal, information about at least one status of the drug delivery device.

## Description

The present invention relates to an electronic module connectable to a drug delivery device and to a drug delivery device for drug administering with such an electronic module.

Drug delivery devices are well known and widely used by patients for self-administered drug injection. However, it has been found that there exists a desire for a highly functional and reliable surveillance of the drug administering process. Such a surveillance is not only relevant for the patients themselves but also in particular during clinical tests to monitor the injection behaviour of the test patients reliably. Also, it is of particular interest to support and guide patients during the usage of drug delivery devices, such that the safety and the adherence of the patient to a prescribed therapy or treatment can be increased.

Furthermore, the integration of an electronic module enabling for said surveillance of the drug administering process should not lead to a significant change of the mechanical handling of the drug delivery device while at the same time, the system or set should be as sustainable, compact and economical as possible.

Therefore, it is an object of the invention to provide an electronic module, a drug delivery device and a set comprising an electronic module and a drug delivery device which is highly reliable, easy to handle, compact, sustainable and economical and which improves safety and therapy adherence, respectively.

This object is solved by the subject matters of the independent claims. Preferable developments are given in the dependent claims.

An electronic module for a drug delivery device in accordance with the present invention comprises a distance sensor configured to generate a signal indicating a distance between the distance sensor and at least one component of the drug delivery device connected to the electronic module, and a processing unit configured to receive the generated signal from the distance sensor and to acquire, based on the received signal, information about at least one status of the drug delivery device.

The acquiring of the information about the at least one status of the drug delivery device assists the user in handling the device and allows for increased safety and therapy adherence.

Preferably, the distance sensor is different from a time-of-flight sensor. For example, the distance sensor is based on a phase-shift measurement, a triangulation measurement and/or an intensity modulation measurement. The distance sensor may be an ultrasonic sensor, a RADAR sensor, a LIDAR sensor and/or an IR sensor. Such distance sensors are particularly reliable and economical.

Preferably, the distance sensor is positioned on a proximal side of the electronic module facing the attached drug delivery device.

The distance sensor may be a contactless distance sensor configured to emit a probe signal towards the component of the drug delivery device and to receive a reflection of the probe signal as a measurement signal from the drug delivery device.

The information about the at least one status of the drug delivery device may comprise at least one of information about whether the drug delivery device is new or has already been used, information about whether the mounting has been successful, a date of the successful mounting, a time of the successful mounting, a number of mounting attempts, an amount of a dose having been set during the dose dialing, a date of the dose dialing, a time of the dose dialing, of information about whether a dose has been dispensed, such as completely dispensed, during the dose dispensing, an amount of a dose having been dispensed during the dose dispensing, a date of the dose dispensing and a time of the dose dispensing.

Another aspect of the present invention provides a drug delivery device comprising a holder for holding the electronic module.

The electronic module may be attachable to and detachable from the drug delivery device or it may be permanently fixed to the drug delivery device via the holder.

Preferably, the component of the drug delivery device and the holder are configured to perform at least a linear movement relative to each other upon the dose dialing and/or dose dispensing.

The component may comprise a plunger rod of the drug delivery device or the component of the drug delivery device is a housing of the drug delivery device.

The distance between the distance sensor and the component of the drug delivery device may be a distance between the distance sensor and a surface of the component, in particular a surface at a distal end of the component facing the distance sensor.

Preferably, at least a part of the surface of the component is configured to reflect a probe signal of the distance sensor as a measurement signal to be received by the distance sensor, wherein, for example, at least a part of the surface of the component comprises a conductive and/or reflective section configured to produce the measurement signal, such as an electromagnetic echo, which can be received by the distance sensor for determining the distance between the distance sensor and the surface of the component, the conductive and/or reflective section being a conductive and/or reflective section cover disposed on the surface of the component or the conductive and/or reflective section being an integral part of the component.

An interior of the drug delivery device between the holder and the component may be free from components interacting with the probe signal and/or the measurement signal such that the probe signal and/or the measurement signal can travel unhindered between the holder and the component.

Alternatively, at least one additional, essentially transparent component may be placed in between the holder and the component, wherein the additional component is configured to absorb and/or reflect the probe signal and/or the measurement signal by less than 10%, such as less than 5%, 2%, 1% or 0.1%.

Another aspect of the present invention provides a set comprising the electronic module and the drug delivery device.

Another aspect of the present invention provides a method of drug delivery comprising generating a signal indicating a distance between a distance sensor of an electronic module and at least one component of a drug delivery device connected to the electronic module, receiving the generated signal from the distance sensor, and acquiring at least one of information about a process of mounting the electronic device on the drug delivery device, information about a dose dialing performed on the drug delivery device and information about a dose dispensing performed on the drug delivery device.

The method may further comprise, before the generating of the signal, determining whether the drug delivery device is new or has already been used. This increases the safety and reliability of the drug delivery process.

The method may further comprise, before the determining whether the drug delivery device is new or has already been used, enabling the dose dialing and/or dose dispensing with the drug delivery device by mounting the electronic component on the drug delivery device. This makes sure that all user interactions are recorded.

An electronic module in accordance with another aspect of the present invention is configured to detect and to monitor a dose administering process with the drug delivery device and/or of parameters related thereto and to provide a user of the drug delivery device with feedback on the conducted dose administering process and/or parameters related thereto. Said electronic module is either integrated with a drug delivery device non-releasably or connectable to a corresponding drug delivery device for self-administered drug injection.

The detection and monitoring of the dose administering process and/or at least of parameters related thereto allows a quite functional and reliable surveillance of the drug administering process. Providing the user with feedback can include feedback information related to the completion and/or success of the administering process. This information can be provided to a user directly during the administering process to guide the user and/or immediately after the end of the drug administering process to inform the user of the success of the administering process. Furthermore, the feedback information can be provided in the form of signals forwarded to a portable device of the user and/or to a server for storing the information for later evaluation. Thus, the electronic module allows highly flexible and reliable surveillance of the drug administering process and, thus, of the administering process with the drug delivery device.

Preferably, the drug delivery device comprises a housing, a cartridge or a syringe filled with a drug to be dispensed and positioned within the housing, a plunger positioned within the cartridge or syringe, a driver positioned within the housing and coupled to the plunger. The driver comprises a drive chassis with a plunger rod and a locked drive spring, wherein the locked drive spring is acting between the housing and the drive chassis and, thus, the plunger rod for generating the force necessary to move the plunger for dispensing the drug. Furthermore, the drug delivery device comprises a needle guard provided axially movable within the housing and configured to release the locked drive spring of the driver for dispensing the drug upon axial movement of the needle guard.

The above structural configuration for a drug delivery device is quite simple and, thus, cost-efficient, while still being highly reliable and secure in use.

Preferably, the electronic module comprises a detection unit and a notification unit. The detection unit is configured to monitor, i.e. to surveil, one or more moving parts of the drug delivery device and to generate information about an administering process of the drug delivery device and/or parameters related thereto based on an operating state of at least one component of the drug delivery device. The notification unit is configured to generate and to output a notification signal including or indicating the generated information about the administering process and/or the parameters related thereto.

Such a configuration was found to be quite favorable for implementing the surveillance or detection, respectively, and the notification in an electronic module.

Further preferably, the detection unit comprises a photo-reflector sensor configured to monitor the movement of at least one component of the drug delivery device, in particular of a drive chassis of the drug delivery device, during the administering process.

A photo reflector sensor depicts a quite reliable and cheap option for implementing the detection functionality.

In such a configuration, the drive chassis comprises preferably at least one optical pattern which is monitored by the photo-reflector sensor for monitoring the dispensing movement of the plunger rod.

This implementation of the movement detection was found to be quite reliable and cost-efficient.

Alternatively, the detection unit comprises preferably a time of flight sensor configured to monitor the movement of at least one component of the drug delivery device, in particular of a plunger rod of the drug delivery device, optionally wherein the plunger rod is a part of a drive chassis of the drug delivery device, during the administering process.

Such a configuration is also a quite cost-efficient but highly reliable option for implementing the detection functionality.

In such a configuration, the time of flight sensor is preferably an optical time of flight sensor and the plunger rod comprises a reflective surface which is monitored by the optical time of flight sensor for monitoring the dispensing movement of the plunger rod.

This specific implementation was found to be of specific reliability and cost efficiency.

Further alternatively, the detection unit comprises a directional microphone, in particular a micro-electromechanical system microphone, configured to detect a signal sound generated by at least one component of the drug delivery device, in particular of a drive chassis of the drug delivery device, during the administering process.

Such a configuration is a further quite cost-efficient but highly reliable option for implementing the detection functionality.

In such a configuration, the drive chassis comprises preferably at least one acoustically active section, in particular a clicker protrusion, generating an acoustic signal upon movement of the drive chassis in the proximal direction, such that the directional microphone can monitor the movement of the plunger rod via the generated acoustic signal.

This specific implementation was found to be of specific cost efficiency.

Further alternatively, the detection unit comprises preferably at least one frangible circuit element configured to be broken when at least one component of the drug delivery device, in particular a drive chassis of the drug delivery device, reaches a specific position during the administering process, to monitor the administering process.

Such a configuration is a further quite cost-efficient but highly reliable option for implementing the detection functionality.

Preferably, the notification unit comprises an active wireless communicator configured to transmit information actively wirelessly to a user device like a smart phone or similar.

Providing a wirelessly communicating notification unit allows to implement highly flexible and connected configurations.

In such a configuration, the active wireless communication can be based on the "cellular" communication technology SigFox.

This communication technology was found to be particularly suitable for implementing the active wireless communication of the electronic module. In particular such a "cellular" communication technology allows the electronic module to transmit data directly to a remote server, without the need for a local intermediate device to relay the information.

Alternatively, the active wireless communication can be based on the "cellular" communication technology NB-loT.

This communication technology depicts suitable alternative of "cellular" communication technology and thus was also found to be particularly suitable for implementing the active wireless communication of the electronic module. However, also other "cellular" communication technologies could be used in the electronic module.

Further alternatively, the active wireless communication can be based on Bluetooth.

Bluetooth based systems are well known and cheaper than the two above discussed "cellular" communication technologies.

Alternative to the above described active wireless communicator, the notification unit can comprise a passive wireless communicator configured to transmit information passively wirelessly to a user device like a smartphone or similar.

Such passive wireless communicators in principle have the advantage over active wireless communicators that they do not need a separate power supply to be operated. This allows to reduce the size and costs of the electronic module.

In such a configuration, the passive wireless communication is preferably based on NFC (near-field communication), in particular with an RFID chip and an antenna.

This specific implementation for the passive wireless communicator was found to be a highly robust and reliable option for implementing the passive wireless communication in the electronic module.

Preferably, the notification unit comprises an optical notifier configured to directly display information to a user of the drug delivery device.

Such an optical notifier allows to provide feedback to a user of the drug delivery device directly without the necessity of any further electronic devices. This direct optical feedback can be given not only after the completion of the drug administering process but also during the administering process to guide the user.

In such a configuration, the optical notifier can include at least one, in particular several, notification LED.

Notification LEDs have been found to depict a quite cheap and suitable option for implementing the optical notifier.

In configurations with an active wireless communicator and/or an optical notifier, the electronic module comprises preferably a micro-controller and a battery. Said micro-controller is configured to receive the information about the administering process from the detection unit, to process the received information and to control the operation of the notification unit to output a user feedback based on the processed information.

Such a configuration is believed to be quite reliable but still simple and, thus, cheap to implement.

Further preferably, the electronic module comprises preferably a, in particular mechanical, wake switch. Said wake switch is coupled to the micro-controller and configured to switch the operation state of the micro-controller between an ON state and an OFF state.

The provision of such a wake switch allows to increase the operable lifetime of the electronic module as the electronic components of the electronic module is switch off as long as the electronic module or the drug delivery device, respectively, is not in use. Thus, the depletion of the provided power supply is minimized during non-usage of the drug delivery device and, thus, of the electronic.

In such a configuration, the wake switch is preferably configured not to be operated by a user of the drug delivery device but automatically during the usage of the drug delivery device, in particular either by the needle guard or by the drive chassis with the plunger rod.

This implementation allows on the one hand a reliable prevention of unintentional actuation of the components of the electronic module and thus of an unintentional depletion of the power supply. On the other hand, it is ensured that the user cannot miss the activation of the electronic module in time to survey the drug administering process.

Preferably, the electronic module is provided as integral component of the drug delivery device non-releasably coupled to the other components of the drug delivery device.

Such an implementation results in quite reliable surveillance of the administering process without the necessity of handling the drug delivery device and the electronic module as two independent but interacting devices. Thus, the handling is simplified substantially.

In such a configuration, the components of the electronic module are preferably positioned within the housing of the drug delivery device.

This allows to implement a quite compact and robust overall configuration.

In particular, the electronic module is positioned side by side with the components of the drug delivery device.

Such a configuration allows to prevent an increase in the length of the drug delivery device as compared to drug delivery devices not provided with the electronic module.

Alternatively, the electronic module can be provided as separate add-on module configured to be coupled releasably to the drug delivery device.

Thus, it would be possible to use one single electronic module together with various drug delivery devices one after another without the necessity of disposing the electronic module. This allows not only a quite environmentally friendly but also relatively cheap surveillance of various administering processes with various drug delivery devices.

In such a configuration, the electronic module comprises preferably a sealing membrane positioned between the electronic module and the drug delivery device in a state in which the electronic module is coupled to the drug delivery device.

Such a sealing membrane allows to prevent the intrusion of dirt or water from the environment of the drug delivery device into the connection between the electronic module and the drug delivery device, and thus possible problems related to such pollution.

In particular, the said sealing membrane is made of material that is transparent for specific light, in particular IR light, such that an optical time of flight sensor can be used for monitoring the administering process through the sealing membrane.

It was found that the usage of such an optical time-of-flight with the add-on on electronic module would depict a quite reliable and cost-efficient implementation, while the transparent sealing membrane ensures the operability of the electronic module.

Preferably, such an add-on electronic module comprises an attachment switch configured to detect the attachment of the electronic module to the drug delivery device.

Thus, the electronic module is configured to detect automatically the connection to the corresponding drug delivery device, which simplifies the handling sufficiently.

In particular, the drug delivery device provided for the usage with such add-on electronic module is provided with an NFC tag containing information about the drug stored within the drug delivery device.

Such an implementation allows an automatic readout of the information about the drug stored within the drug delivery device and, thus, allows to increase the functionality of the electronic module.

In such a case, the electronic module comprises preferably an NFC reader configured to automatically read the information stored within the NFC tag.

Thus, the information about the drug stored within the drug delivery device can be read out automatically by the electronic module.

Preferably, at least one component of the drug delivery device comprises a barcode or QR code containing information about the drug within the drug delivery device and configured to be read by a user device like a smartphone.

Such a barcode or QR code is a quite simple but efficient way to provide information about the drug delivery device to a user device.

Preferably, an add-on electronic module comprises a mechanical coding feature only allowing the attachment of the electronic module to drug delivery devices provided with a matching mechanical coding feature.

Thus, a dedication of an electronic module to specific drug delivery devices is implemented in a quite cost-efficient but reliable manner.

Preferably, the electronic module is configured to be positioned or is positioned at a distal end of the drug delivery device axially behind the other components of the drug delivery device.

Thus, the width of the drug delivery device is not increased by the electronic module. Accordingly, the handling of the drug delivery device is less influenced by the provision of the electric module.

Preferably, the drug delivery device is a single use fixed dose drug delivery device.

Such drug delivery devices are structurally quite simple and, thus, quite cheap.

Alternatively, the drug delivery device is a single use variable dose drug delivery device.

Such drug delivery devices are more flexible in use then fixed dose drug delivery devices.

Further alternatively, the drug delivery device is a multiple use fixed dose drug delivery device.

Such drug delivery devices are structurally quite simple but with the multiple use configuration more environmentally friendly than the single-use drug delivery devices.

Finally, the drug delivery device can be a multiple use variable dose drug delivery device.

Such an implementation provides the most flexibility and environmental friendliness.

Preferably, the electronic module is configured to detect and to monitor the dose administering process with the drug delivery device or parameters related thereto based on a movement status of one or more moving parts of the drug delivery device or based on a position of one or more moving parts of the drug delivery device.

Such implementations were found to result in quite reliable results for the surveillance of the administering process.

Alternatively, the electronic module is configured to detect and to monitor the dose administering process with the drug delivery device or parameters related thereto based on both of a movement status and a position of one or more moving parts of the drug delivery device.

The combined movement and position detection results in the most reliable results for the surveillance of the administering process.

In such a configuration, the electronic module is preferably configured to detect and to monitor the dose administering process with the drug delivery device or parameters related thereto based on the movement status and the position of one single moving part of the drug delivery device, in particular a drive chassis with the plunger rod.

The monitoring of one single moving part it is rather simple and thus cost-efficient, while allowing still reliable surveillance of the administering process. The drive chassis with the plunger rod is the component identified to depict the best option for such as single part of the drug delivery device as its movement is directly connected to the administering process.

Preferably, the electronic module is configured to wait a specific period of time before finally determining the end of the dose administering process after no further change in any one of the monitored parameters is determined any more.

Such a time delay was found to be as simple option for ensuring that the administering process is completed.

In particular, the specific period of time is 1, 2, 3, 5 or 10 seconds.

Said values for the specific period of time have been found to be specifically suitable.

Preferably, wherein an outer contour of the drug delivery device together with the electronic module is flush seen along the longitudinal axis of the drug delivery device.

It was found that such a flush outer contour would result in an improved handling of the overall system.

With all aspects and embodiments, the drug delivery device may be an autoinjector.

Exemplary embodiments and functions of the present disclosure are described in the following in conjunction with the appending drawings, wherein:
- FIG. 1: shows a cross-sectional view of a drug delivery device with a basic injection configuration of an exemplary single-use fixed-dose drug delivery device to be used with the electronic module of the present disclosure;
- FIG. 2A: shows a cross-sectional view of the drug delivery device of FIG. 1 as delivered to user;
- FIG. 2B: shows a cross-sectional view of the drug delivery device of FIG. 1 directly before drug delivery;
- FIG. 2C: shows a cross-sectional view of the drug delivery device of FIG. 1 after completion of the administering process;
- FIG. 3: shows a cross-sectional view of a first exemplary embodiment for a drug delivery device with an electronic module according to the present disclosure;
- FIG. 4: shows enlarged and turned sectional view of the drug delivery device of FIG. 3;
- FIG. 5A: shows further details of the drug delivery device of FIGS. 3 and 4 before drug dispensing;
- FIG. 5B: shows further details of the drug delivery device of FIGS. 3 and 4 during drug dispensing;
- FIG. 6: shows diagrams describing the function of the wake switch of the drug delivery device of FIGS. 3 to 5B;
- FIG. 7: shows a cross-sectional view of a second exemplary embodiment for a drug delivery device with an electronic module according to the present disclosure;
- FIG. 8: shows an enlarged and turned sectional view of the drug delivery device of FIG. 7;
- FIG. 9: shows a cross-sectional view of a third exemplary embodiment for a drug delivery device with an electronic module according to the present disclosure;
- FIG. 10A: shows a cross-sectional view of a fourth exemplary embodiment for a drug delivery device, configured to be connected with an add-on electronic module, in accordance with the present disclosure;
- FIG. 10B: shows a cross-sectional view of the drug delivery device of FIG. 10A coupled with an exemplary add-on electronic module in accordance with the present disclosure;
- FIG. 11: shows an enlarged and turned view of the electronic module of FIG. 10B;
- FIG. 12: shows symbolically two exemplary wireless drug identification configurations in accordance with the present disclosure;
- FIG. 13: shows symbolically a further exemplary wireless drug identification configuration in accordance with the present disclosure;
- FIG. 14: shows an exemplary embodiment of a mechanical drug identification configuration;
- FIG. 15: shows a set comprising an electronic module and a drug delivery device in accordance with the disclosure;
- Fig. 16: shows a further embodiment of the set of Fig. 15;
- Fig. 17: shows a perspective view of a fifth exemplary embodiment of a drug delivery device and electronic module according to the present disclosure;
- Fig. 18: shows a cross-sectional side view of the fifth embodiment with the drug delivery device in an initial and end of dose status;
- Fig. 19: shows a cross-sectional side view of the fifth embodiment with the drug delivery device in a set dose status;
- Fig. 20: shows a cross-sectional side view of a sixth embodiment of an electronic module for the drug delivery device shown in Figs. 17 to 19;
- Fig. 21: shows a further cross-sectional side view of the sixth embodiment of the electronic module for the drug delivery device shown in Figs. 17 to 19 in a plane perpendicular to the plane of Fig. 20;
- Fig. 22: shows a schematic diagram of the sixth embodiment of the electronic module attached to the drug delivery device;
- Fig. 23: shows a sense circuit of the drug delivery device for the sixth embodiment of the electronic module in an initial and end of dose status in a first perspective view;
- Fig. 24: shows the sense circuit with the drug delivery device for the sixth embodiment of the electronic module in the initial and end of dose status in a second perspective view;
- Fig. 25: shows the sense circuit with the drug delivery device for the sixth embodiment of the electronic module in the activated status with a dose set in a perspective view;
- Figs. 26 to 28: show a holder of the drug delivery device for mounting the electronic module to the drug delivery device of the sixth embodiment;
- Figs. 29 to 30: show an electrical connector of the drug delivery device for the electronic module of the sixth of embodiment;
- Fig. 31: shows a first lock for the drug delivery device shown in Fig. 1;
- Fig. 32: shows a second lock for the drug delivery device shown in Fig. 1.

In this disclosure, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the members thereof, which in accordance with the use of the device is located closest to the delivery/injection site of the patient.

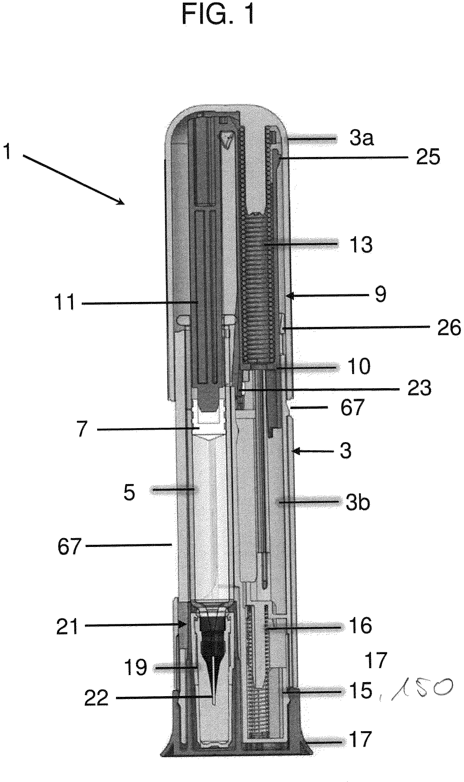

The present disclosure of administering process surveillance is applicable with a number of drug delivery devices. One possible drug delivery device is a pen-type designed drug delivery device with the basic injection configuration as illustrated in FIG. 1.

FIG. 1 shows an exemplary embodiment of a drug delivery device 1 configured to be supplemented by or configured to be used together with an electronic module 27 according to the present disclosure.

The drug delivery device 1 comprises a housing 3 formed of an outer body 3a and an inner body 3b. A cartridge or syringe 5 filled with a drug to be administered is positioned within the housing 3. A plunger 7 for dispensing the drug from the cartridge or syringe 5 is positioned within the cartridge or syringe 5. Furthermore, a driver 9 is positioned within the housing 3 and coupled to the plunger 7. Said driver 9 comprises a drive chassis 10 with plunger rod 11 and a locked drive spring 13 acting between the housing 3 and the drive chassis 10 with the plunger rod 11 for generating the force necessary to move the plunger 7 through the cartridge or syringe 5 for dispensing the drug. Moreover, the illustrated drug delivery device 1 comprises a needle guard 15 movable against the force of a needle guard spring 16 from a proximal end of the drug delivery device 1 in the distal direction into the housing 3. Said needle guard 15 is coupled to the drive chassis 10 in such a manner that a distal movement of the needle guard 15 releases the locked drive spring 13 resulting in a proximal movement of the plunger rod 11 and, thus, of a dispensing movement of the plunger 7 along the cartridge or syringe 5.

For the sake of security, the drug delivery device 1 further comprises a cap 17 with a rigid needle shield 19 connected to the proximal end of the drug delivery device 1. This cap 17 not only shields the needle 22 but also prevents a distal movement of the needle guard 15.

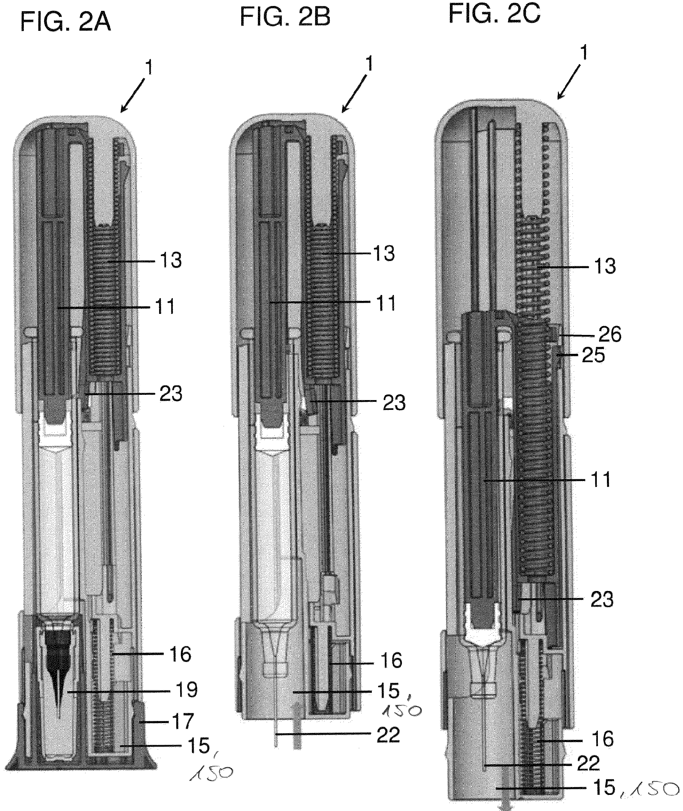

In the following, the basic function of the illustrated drug delivery device 1 will be described with reference to FIGS. 2A to 2C.

As can be seen in FIG. 2A, in the delivery state of the drug delivery device 1 the cap 17 is positioned on the proximal end of the drug delivery device 1. The rigid needle shield 19 of this cap 17 encloses a needle assembly 21 with the needle 22 of the cartridge or syringe 5. The drive chassis 10 with the plunger rod 11 and the drive spring 13 are in an initial state, in which the drive spring is loaded and locked via a locking element 23 being engaged with the housing 3. Thus, the drug delivery device 1 is in a pre-filled and preloaded status.

For the drug administering, the cap 17 together with the needle shield 19 is removed from the proximal end of the drug delivery device 1. Then, the drug delivery device 1 is pressed with the needle guard 15 at its proximal end against an injection site (not illustrated). This results in a distal movement of the needle guard 15 with respect to the housing 3, while the needle 22 of the needle assembly 21 penetrates the injection site. Near a distal end position of the needle guard 15, the needle guard 15 disengages the locking element 23 of the drive chassis 10 from the housing 3 and, thus, releases the drive spring 13. Exactly this situation is illustrated in FIG. 2B.

The needle guard 15 forms a movable trigger 150 that displaces the locking element 23 by an unlocking movement to initiate dose delivery. With the drug delivery device 1, the drive chassis 10 forms a first component that is configured to perform a movement with respect to a second component formed by the housing 3 during the dose delivery operation. The locking element 23 thereby locks the first component 10 to the second component 3 prior to dose delivery. The trigger 150 formed by the needle guard 15 is configured to displace the locking element 23 by an unlocking movement to initiate dose delivery.

The released drive spring 13 pushes the drive chassis 10 with the plunger rod 11 in the proximal direction, which results in a corresponding movement of the plunger 7 and, thus, in a dispensing of the drug from the cartridge or syringe 5 through the needle 22 into the injection site. After this dispensing, the drug delivery device 1 is removed from the injection site. This results in the needle guard spring 16 pushing the needle guard 15 in the proximal direction such that the needle guard 15 encloses the needle 22. In this extended state, an acoustically active section 25 of the drive chassis 10 engages with an end of dose click protrusion 26 of the housing 3 generating an end of dose click and a locking of the movement of the components of the driver 9 with respect to the housing 3. Moreover, the locking element 23 of the drive chassis 10 engages with the needle guard 15 preventing a further distal movement of the needle guard 15 with respect to the housing 3. This finally locked state is illustrated in FIG. 2C.

In this connection it should be noted that the drive chassis 10 is arranged linearly moveable within the housing 3. The drive chassis 10 comprises a dispensing limb forming the plunger rod 11 and a trigger limb within which the drive spring 13 is arranged. The plunger rod 11 is arranged at a proximal end of said dispensing limb and the locking element 23 is arranged extending proximally from said trigger limb. The trigger limb and the dispensing limb are arranged in parallel to one another respectively at least essentially in parallel to one another and are connected to one another, preferably are integrally formed in one piece, at a respective distal end of the dispensing limb and the trigger limb. This shape of drive chassis 10 enables a particularly compact design of the drug delivery device 1.

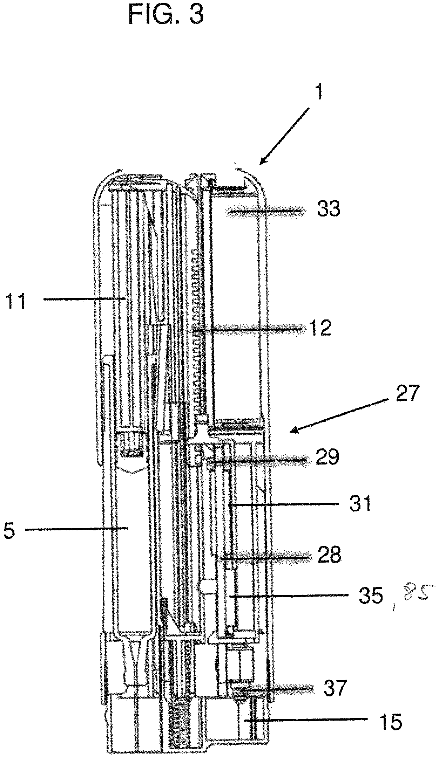

In the following, a first exemplary embodiment for a drug delivery device with an integrated electronic module in accordance with the present disclosure will be described with reference to FIGS. 3 to 6.

The basic injection configuration of the here illustrated drug delivery device 1 is in principled identical with the one of the drug delivery device 1 described with reference to FIGS. 1 to 2C.

The above described drug delivery device 1 is merely supplemented with an exemplary electronic module 27 in accordance with the present disclosure. Said electronic module 27 is positioned side by side with the above described components of the drug delivery device 1 within the housing 3, in particular within the outer body 3a of the housing 3.

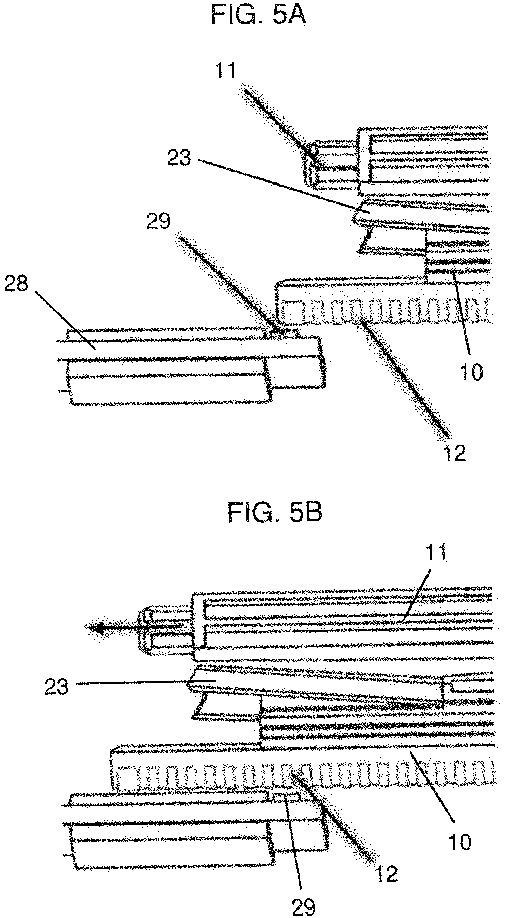

The electronic module 27 comprises a printed circuit board arrangement (PCBA) 28 including a detection unit with a photo-reflector sensor 29 and a micro-controller 31 as well as a power supply 33 in the form of a battery, in particular of a standard AAA size battery (preferably either alkaline or LiMnO₂). The micro-controller 31 forms a processing unit of the electronic module 27. The micro-controller 31 is coupled to an active wireless communicator 35 in the form of an NB-loT module of a notification unit of the electronic module 27. The communicator 35 forms an exemplary embodiment of a communication unit 85 of the electronic module 27. Furthermore, a wake switch 37 (described later in more detail) is provided in the proximal end of the housing 3 and coupled to the micro-controller 31. Finally, also an optical notifier in the form of a notification LED (not illustrated) is connected to the micro-controller 31. The drive chassis 10 is provided, in particular printed, with an optical pattern 12 in the form of a printed coded track with sections of differing reflectivity. This is illustrated in FIGS. 5A and 5B, where FIG. 5A shows the relative positioning between the optical pattern 12 and the photo-reflector sensor 29 immediately before the release of the drive spring 13 and FIG. 5B shows the relative positioning between the optical pattern 12 and the photo-reflector sensor 29 at a specific point of time during the dispensing movement of the drive chassis 10 with the plunger rod 11.

The photo-reflector sensor 29 is positioned such that the region of the drive chassis 10 with the optical pattern 12 passes the photo-reflector sensor 29 during a dispensing movement of the drive chassis 10 with the plunger rod 11 in the proximal direction. Thus, the photo-reflector sensor 29 is configured to monitor the dispensing movement of the plunger rod 11 with respect to the housing 3. For this, the photo reflector sensor 29 is coupled non-movably to the housing 3. The micro-controller 31 is configured to receive varying brightness information corresponding to the movement of the drive chassis 10 with respect to the photo-reflector sensor 29 and to process the received information. In particular, the micro-controller 31 is configured to determine both the present position of the drive chassis 10 with the plunger rod 11 within the housing 2 and the present movement status of the drive chassis 10 with the plunger rod 11 for evaluating the current state of the administering process. However, the micro-controller 31 can also be configured to determined only one of the present position of the drive chassis 10 with the plunger rod 11 within the housing 2 and the present movement status of the drive chassis 10 with the plunger rod 11 for evaluating the current state of the administering process, if desired. The micro-controller 31 is further configured to control the operation of the various notification units of the electronic module 27, here in particular of the NB-loT module 35 and of the notification LED to output a user feedback based on the processed information.

In particular, the notification LED is illuminated and switched off in a particular pattern to inform the user of the drug delivery device 1 of the status of the administering process. At the same time or at a later time, the NB-loT module 35 transmits wirelessly electronic information about the administering process to an external server, where this information is stored.

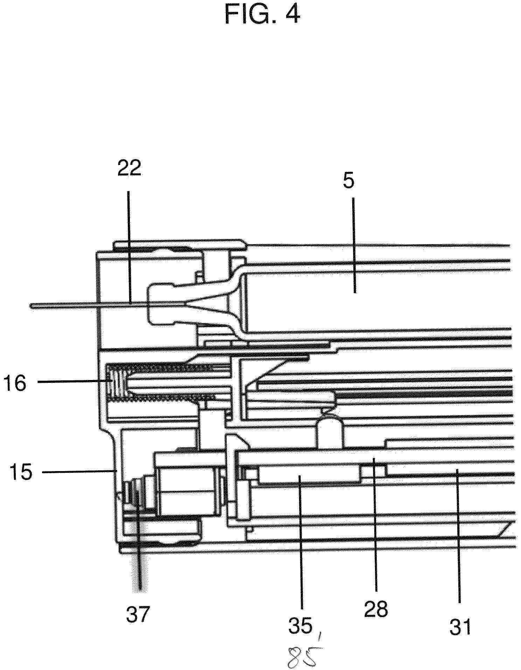

To extend the service life of the electronic module 27, the micro-controller 31 is configured to be in an inactive sleep-state until the wake switch 37 is activated. For this, the wake-switch 37 is positioned in such a manner that it is activated only through a distal movement of the needle guard 15 immediately before the release of the drive spring 13 as can be seen in FIG. 4. The wake switch 37 is a mechanical wake switch 37 due to the minimum energy consumption of such a mechanical switch in comparison to accelerometers or thereto similar components.

The micro-controller 31 is not only configured to notice the activation of the wake switch 37 but also its deactivation thereof upon a proximal movement of the needle guard 15 and a thereto related release of the wake switch 37. In particular, the micro-controller 31 is configured to determine the period of time during which the wake switch 37 is activated and to compare this period of time with a predetermined period of time expected to be necessary for a full administering process. Accordingly, the micro-controller 31 is configured to not only detect and monitor the dose administering process based on the movement of the drive chassis 10 with the plunger rod 11 but also based on the movement of the needle guard 15.

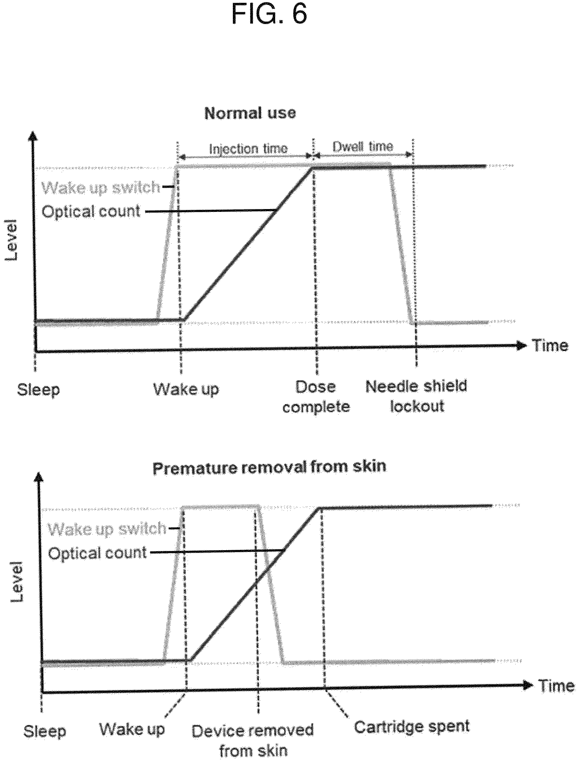

As for example illustrated in FIG. 6, a full administering process would at least require a specific predetermined injection time and a further dwell time from the full activation of the wake switch 37 (see the above diagram). If the wake switch 37 is deactivated before the expiration of this predetermined period of time by a removal of the drug delivery device 1 from the injection site and, thus, by a proximal movement of the needle guard 15, the micro-controller 31 determines an incomplete administering process, although the drive chassis 10 with the plunger rod 11 finishes its whole dispensing movement.

In other words, the use of the needle guard 15 to actuate the wake switch 37 offers the benefit in being able to detect premature removal of the drug delivery device 1 from the injection site before dose delivery has completed.

In other words, during normal operation, the wake switch 37 is actuated before movement of the plunger rod 11 commences, and the wake switch 37 is not released until after movement of the plunger rod 11 has ceased. When the drug delivery device 1 is lifted away from the injection site the needle guard spring 16 will displace the needle guard 15 proximally, into its extended position. The wake switch 37 will register a change in state since the needle guard 15 will no longer be in interference with the wake switch 37. Therefore, if movement of the plunger rod 11 continues after the wake switch 37 is released this indicates premature removal of the drug delivery device 1. In this case, the event may be recorded as an error within the micro-controller 31 to be transmitted alongside the dispense time and drug information. This information may be subsequently used to mitigate future premature removal either through user training or other intervention.

Such a functionality is of particular relevance when the micro-controller 31 is provided with further information about the drug delivery device 1 like the two above referenced time periods. In the present configuration, in which the electronic module 27 is integrated into the drug delivery device 1, these and also other information like the specific drug within the cartridge or syringe 5 can be directly stored in a memory connected to the micro-controller 31.

As indicated above, a full administration of the drug could require a specific dwell time. Thus, if for example the optical notifier LED is used to guide the user through the administering process, the micro-controller 31 of the electronic module 27 could wait a specific period of time before indicating the end of the dispensing process after the monitored component, here the drive chassis 10 with the plunger rod 11, is not moving any more, respectively after the monitored parameter is not changing any more. It has been found that suitable time periods lie between 1 and 10 seconds, depending on the used drug and the structural configuration of the drug delivery device 1 itself.

In principle, there are various aspects of a dose administering process which could be detected and monitored by the electronic module 27. In particular, the start of the dose dispensing and the successful completion of the dose dispensing can be determined. Furthermore, the electronic module 27 can be configured to determine a termination of the dispensing process, for example after no dispensing movement or change in the monitored parameter is determined for a specific time going for example from 1 sec to 10 secs after the start of the dose dispensing was determined but the successful end of the dose dispensing has not been determined yet. Also, the specific development of the administering process could be monitored, in the present embodiment in particular based on the movement characteristics of the drive chassis 10 with the plunger rod 11 with respect to the photo-reflector sensor 29 and, thus, with respect to the housing 3.

For this, the photo-reflector sensor 29 comprises an LED for illumination and a photo transistor for detecting the light reflected from the optical pattern 12 on the drive chassis 10. The optical pattern 12 is formed of more reflective and less reflective regions positioned in view of the photo-reflector sensor 29 such that an axial movement of the drive chassis 10 with the plunger rod 11 results in changes in intensity of reflected light determined by the photo transistor. This signal is transmitted to the micro-controller 31.

Such a sensing system provides a relative measure of the distance travelled by the drive chassis 10 with the plunger rod 11 and allows the micro-controller 31 to determine that the plunger rod 11 has translated far enough for full drug delivery. Furthermore, the micro-controller 31 can determine when movement of the drive chassis 10 with the plunger rod 11 has ceased. The final axial position of the plunger rod 11 to the housing 3 at the end of dispense is subject to large tolerance variation, primarily due to this end stop being defined by the plunger 7 at the proximal end of the cartridge or syringe 5. As a result, if a mechanical switch were used to detect end of dispense, the combined tolerance effect of the device tolerances coupled with the switch point variation and PCB mounting tolerances mean such an alternative system could be too imprecise. By detecting both the axial position of the drive chassis 10 with the plunger rod 11 and whether it has ceased moving allows the micro-controller 31 to more reliably identify the end of the dispensing operation even with relatively large mechanical and electronic detection mechanism tolerances.

If the end of dispense is determined through detecting the axial position of the drive chassis 10 with the plunger rod 11 only, the point at which end of dispense feedback is triggered may be nominally arranged to occur before the plunger rod 11 ceases moving. This ensures that, even when the plunger rod 11 comes short of its nominal axial displacement due to component and system tolerances, the end of dispense feedback is still triggered. By also monitoring the continuing movement of the drive chassis 10 with the plunger rod 11, this information can be combined with the relative displacement of the plunger rod 11 and the end of the dispensing process may be only determined when the drive chassis 10 with the plunger rod 11 has advanced far enough and also ceased moving. This improves the accuracy of the timing of the end of the dispensing process and ensures it more closely aligns with the precise moment that dose delivery completes.

Here, the optical pattern 12 is a printed pattern on the drive chassis 10, since minimal changes to the original drug delivery device mechanism (see FIGS. 1 to 2C) are preferred. However, functionally, this optical pattern 12 could be also formed by moulded-in features/recesses, laser surface markings, a separate component or other similar techniques. In addition, other sensing technologies, such as coded magnets or capacitance sensors could be employed to provide similar feedback of the plunger rod 11 movement.

In the present embodiment, the successful completion of the dose administering process is determined when the drive chassis 10 with the plunger rod 11 has reached full proximal displacement and has ceased moving. The micro-controller 31 is configured to identify a stall (either due to mechanism or PFS failure), where the plunger rod 11 stops moving completely or moves so slowly that it does not achieve full proximal displacement within a pre-determined time limit. This would be recorded as an error event.

On dispense completion the system attempts data transmission to a remote system (the 'cloud') using the NB-loT or similar network. Optionally, the data transmission may not start until the wake switch 37 has also been released, indicating that the needle guard 15 has extended as the drug delivery device 1 is removed from the injection site. The data transmitted may include variables relating to the dose administering process such as the time since wake, time since dispense completion, time since error state and displacement of the drive chassis 10 with the plunger rod 11, device status flags such as detection of a stall, premature removal, low battery etc. alongside drug specific information such as type, concentration, batch and expiry date, alongside data that can be used to link the device to the patient to which it was prescribed.

The one or more notification LEDs, visible to the user through a cut-out, transparent window or thin region in the housing 3, provide visual feedback to the user during use of the drug delivery device 1. In particular, different LED flashing patterns and/or colors may be used to indicate the status of the drug delivery device 1 to the user. Examples for the status is whether the wake switch 37 is activated, dispense is in progress, the dose has been successfully delivered, an error state has been detected, data transmission is in progress and whether data has been successfully transmitted.

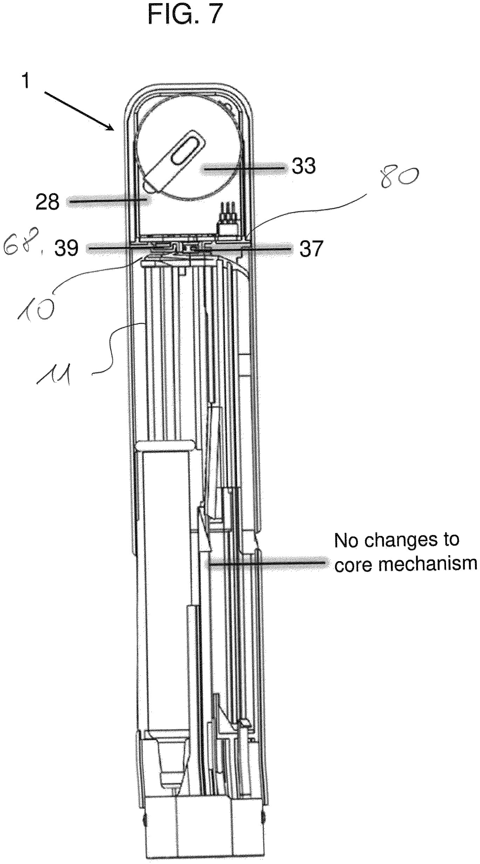

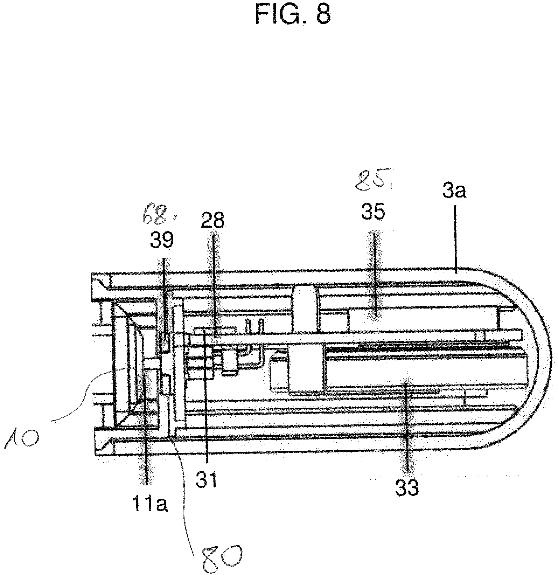

In the following, a second exemplary embodiment of a drug delivery device 1 with a corresponding electronic module 27 will be described with reference to FIGS. 7 and 8. As far as no differences are disclosed, the second exemplary embodiment is configured as it is disclosed for the first exemplary embodiment and vice versa.

Here, the electronic module 27 is provided as integrated module provided within a distal end of the housing 3 behind the other components of the drug delivery device 1. The outer body 3a of the housing 3 is increased in length to accommodate the power supply 33 and other electronics mounted in series with the injection mechanism to facilitate the connectivity features.

No changes are required to the core principles and the majority of the components of the injection mechanism of the drug delivery device 1 as illustrated in FIGS. 1 to 2C.

The electronic module 27 in this embodiment substantially comprises a PCBA 28 with a micro-controller 31, an active wireless communicator 35 in the form of a SigFox module, a distance sensor in the form of a time of flight sensor 39, a wake switch 37, a notification LED (not illustrated) and a power supply 33.

Due to the reduced space, the power supply 33 exemplarily is a relatively compact coin cell (high drain CR2032) which have been specifically developed to meet the requirements of the SigFox communication technology. With other embodiments, the power supply 33 could also be of other type. The present cell 33 is mounted using a proprietary coin cell holder. As a result, this integrated option necessitates relatively minor changes in the overall configuration of the drug delivery device 1, in particular adding about 27 mm in length and about 1.6 mm in width.

Given the integrated nature of this drug delivery device 1, the drug information can be coded directly into a memory of the micro-controller 31 during manufacture and/or assembly. This avoids the need for the electronic module 27 to have the capability to identify different medicaments assembled into the drug delivery device 1 and allows this information to be transmitted on completion of the dose administering process.

In the following, the function of the present drug delivery device 1 will be described roughly.

Prior to dispense the distal end of the plunger rod 11 interacts with the wake switch 37, holding the wake switch 37 in a depressed state. While this wake switch 37 remains pressed, the electronic module 27 remains in a very low power deep sleep state, maximizing battery life of the drug delivery device 1.

As dispense begins, the plunger rod 11 moves proximally, releasing the wake switch 37. This signal initiates the electronic module 27 to bring the micro-controller 31 out of a deep sleep state. In order to achieve the necessary long storage time for these types of devices, the wake switch 37 is a mechanical detect switch
and in this case is expected to be a normally closed type (such that when the switch is released, a circuit is completed). In the storage condition therefore, the circuit remains open, so this does not draw quiescent current and allows the micro-controller 31 to remain in a very low energy state until dose dispense starts. Other active sensing options, such as accelerometers draw current themselves, and require the micro-controller 31 to be in a higher power monitoring state throughout the drug delivery device 1 storage life prior to drug dispensing.

During dose dispensing the plunger rod 11 continues to translate axially in the proximal direction until the end of dose is reached. It is preferable to detect dose completion in addition to simply the start of a dose administering process to provide positive confirmation that the dose was successfully delivered in full. If the dose administering process is not completed successfully, for example due to mechanism stalling or another adverse event, this can be identified and feedback can be provided to the user or healthcare professional.

A number of different non-contact methods for detecting the end of the administering process are possible. In the present embodiment, a distance sensor configured as a time of flight sensor 39 is used. The present time of flight sensor 39 is an optical time of flight sensor 39 emitting a pulse of infrared light and detecting the time taken for this pulse to be reflected back to an adjacent detector of the sensor 39. The time taken is used to calculate the distance between the sensor 39 and the target.

In this embodiment, the time of flight sensor 39 is positioned to target a distal and reflective surface 11a of the plunger rod 11. During dispense, the distance between the time of flight sensor 39 and the plunger rod 11 increases, until the plunger rod 11 ceases moving on dose dispensing completion. Once the time of flight sensor 39 detects both that the plunger rod 11 is stationary and that it has translated axially far enough to achieve full dispense, the micro-controller 31 records the completion of the dose dispensing process. With other embodiments, the completion of the dose dispensing process may also be detected by only detecting that the plunger rod 11 has translated axially far enough to achieve full dispense. Detection whether the piston rod 11 has translated far enough may comprise comparing a distance between the time of flight sensor 39 and the movable component formed by the plunger rod 11.

This sensing system provides a relative measure of the distance travelled by the plunger rod 11 and allows the micro-controller 31 to determine that the plunger rod 11 has translated far enough and when movement of the plunger rod 11 has ceased.

Alternatively, also a directional microphone 39, in particular a MEMS (micro-electromechanical system) microphone system activated by release of the wake switch 37 can be used instead of the time of flight sensor 39.

When the plunger rod 11 approaches the end of dispense, a characteristic audible click is created by a resilient feature 25 of or on the drive chassis 10 interacting with at least one corresponding feature 26 of the housing 3. Such a resilient feature 25 serves as acoustically active section 25 of the drive chassis 10 and in particular is provided in the form of a clicker protrusion. This is expected to create a specific frequency and amplitude of sound that, via signal processing conducted by either the micro-controller 31 or a separate dedicated micro-processor, can be differentiated from other background noise.

To improve the robustness of the system, multiple distinctive clicks may be generated by the mechanism, all or some of which must be identified by the electronic module 27 before the end of the dose administering process is confirmed. In addition to detecting the end of dose dispensing, the MEMS microphone system could also be used to identify when the needle guard 15 advances, if this is accompanied by another distinguishable sound. This could be used to inform about a correct sequence of operation, in a similar way as the wake switch 27 is employed for premature removal detection as described above. This has the benefit of avoiding the addition of significant measurement related tolerances.

Other non-contact methods of detecting the end of dispense (for example
accelerometers, ultrasonic sensors, camera-based vision system etc.) may also be realized with other embodiments.

The data transmission to the user and/or other devices in principle is identical to the one described for the first embodiment. The only difference is that compared to the NB-loT network, the SigFox network is limited in its ability to provide positive confirmation that the transmitted information has been successfully received by the remote system. As a result, the SigFox based electronic module 27 may be configured to repeatedly attempt transmission of the data following dispense for a predefined number of iterations or until the power supply 33 is exhausted to maximize the chance of successfully uploading the data.

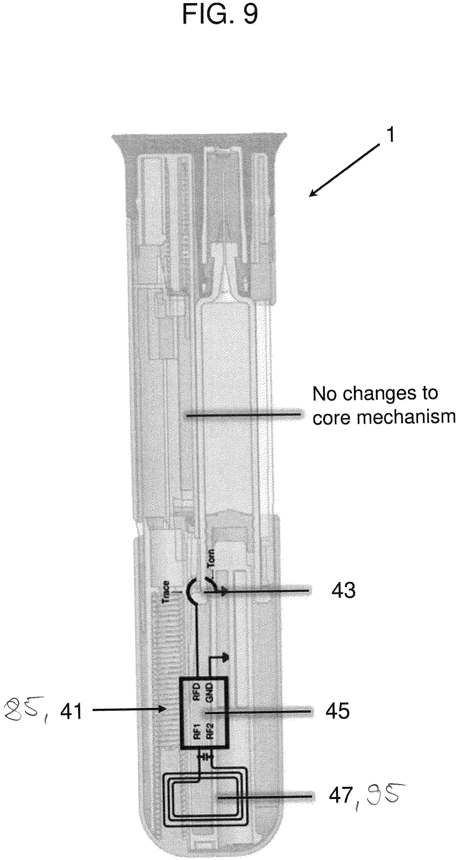

In the following, a third exemplary embodiment for a drug delivery device 1 with an electronic module 27 in accordance with the present invention will be described with reference to FIG. 9. As far as no differences are disclosed, the third exemplary embodiment is configured as it is disclosed for the first and second exemplary embodiments and vice versa.

This embodiment requires merely minimal changes to the core mechanism of the drug delivery device 1 as described with reference to FIGS. 1 to 2C. Here, the detection or monitoring of the drug administering process and the notification of the user is achieved by an integrated NFC connectivity system 41.

In contrast to the above described "cellular" communication integrated systems, the NFC integrated option does not include a power supply 33. For NFC systems, that utilize RFID (radio-frequency identification) technology, the circuitry integrated into the drug delivery device 1 serves as passive wireless communicator and may not require a built-in power supply 33. In general, the NFC connectivity system 41 forms a communication unit 85 of the device. When a smartphone with NFC capability is brought into close contact with the integrated NFC drug delivery device 1, the magnetic field for example created by a smartphone can be used to provide power to the passive NFC tag built into the drug delivery device 1.

The illustrated integrated NFC option uses an NFC circuit arranged with a frangible circuit element 43, in particular in the form of a loop of wire, that is broken at the end of the dispensing process by the drive chassis 10. Before use, when a smartphone is brought into close contact with the integrated NFC drug delivery device 1, the RFID chip 45 is energized via an antenna 47 and acts as a transponder, providing a specific response to the smartphone to indicate that the drug delivery device 1 has not yet been used.

Then, the drug delivery device 1 may be activated by a user to dispense the dose without any modification to user steps or specific instructions related to the connectivity system. As the drive chassis 10 with the plunger rod 11 advances proximally during the administering process, it interacts with the frangible loop of wire 43 connected to the RFID chip 45, breaking it as the end of the dispensing process is reached. The breakage of the wire 43 alters the signal that is transmitted by the RFID chip 45 when energized by the smartphone after use so that it can be confirmed that the drug delivery device 1 has been used.

It is preferable to arrange the frangible circuit element 43 such that it is broken as near to the end of the dispense stroke of the drive chassis 10 with the plunger rod 11 as possible. In this way, the change of state indicated by the breaking of the element 43 can be used to provide a positive confirmation that the dose was successfully delivered in full.

If the administering process is not completed successfully and the drive chassis 10 with the plunger rod 11 does not proximally translate fully, due to mechanism stalling or another adverse event, the frangible circuit element 43 will not be broken and the drug delivery device 1 will not report dose completion.

Drug and/or device information can be programmed into a part of the RFID circuit not affected by the breakage of the frangible circuit element 43, such that this can be read both before and after use of the drug delivery device.

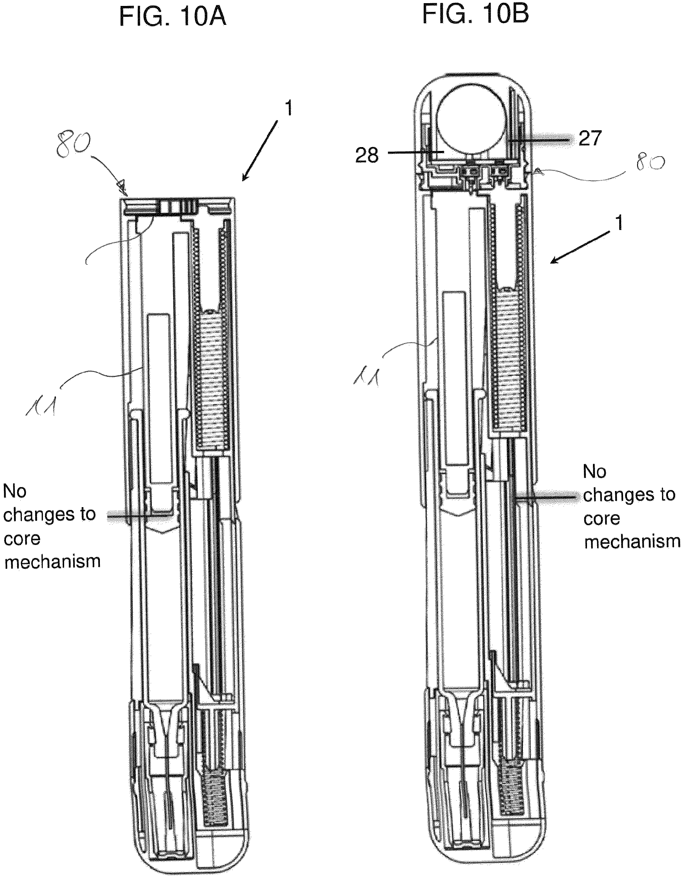

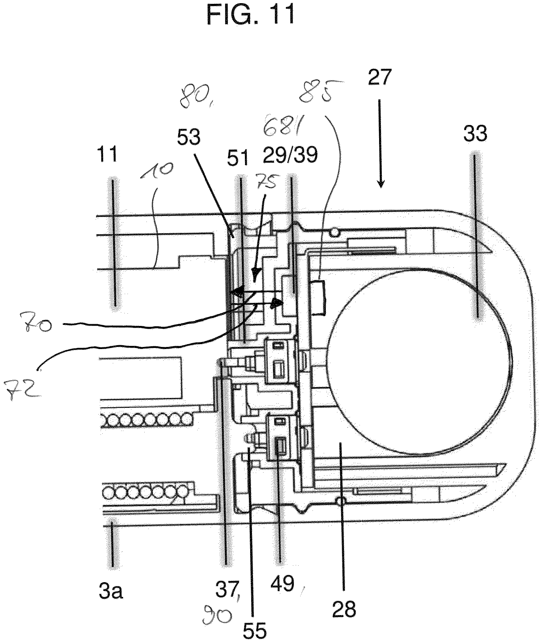

In the following, a fourth exemplary drug delivery device 1 with an electronic module 27 in accordance with the present invention will be described with reference to FIGS. 10A, 10B and 11. As far as no differences are disclosed, the fourth exemplary embodiment is configured as it is disclosed for the first, second and third exemplary embodiments and vice versa.

Here, the electronic module 27 is provided as separate add-on module coupled releasably to a holder 80 provided at the distal end of the drug delivery device 1. Such an add-on configuration provides the opportunity to re-use the electronic module 27 for multiple doses with multiple different drug delivery devices 1. With the first, second and third embodiment, the holder 80 to hold the electronic module 27 is integrated into the drug delivery device 1 and the electronic module 27 is non-releasably held by the holder 80.

Only minor modifications to the housing 3 allow a design of the drug delivery device 1 to be used both with or without the add-on connected module 27.

The connected electronic module 27 of the present embodiment substantially comprises a PCBA 28 with a processing unit formed by a micro-controller 31, a dispense sensor 91 or 29 or 39, a wake switch 37, an attachment sensor formed by an attachment switch 49, a user notification LED (not illustrated) and a power supply 33. Furthermore, a communication unit 85 exemplarily allowing for an active wireless communication configuration based on Bluetooth is provided. The lower peak currents involved in Bluetooth transmissions allow for a smaller coin cell to be used as compared to the above described integrated "cellular" options. This allows a quite compact add-on module 27. Such an electronic module 27 can be accommodated within the width and depth of the existing housing 3 of the implementation described with reference to FIGS. 1 to 2C and has a length of approximately 18 mm.

A sealing membrane 51 is provided on the proximal end of the electronic add-on module 27 to prevent ingress of fluids and particles which may affect function of the add-on module 27. The sealing membrane 51 is flexible to enable the wake switch 37 and the attachment switch 49 to operate by deflection of locally thinned or convoluted regions of this sealing membrane 51. In the case where an optical time of flight sensor 39 is employed, the sealing membrane 51 is at least partly also made of material that is transparent for light of specific wavelengths, in particular to IR light, permitting the correct function of the sensor 39.

Like the sensor 39 of the second embodiment, the sensor 39 of the fourth embodiment is configured as a distance sensor 68 and serves the same functions as the distance sensor 39 of the second embodiment. The distance sensor 68 is configured as a contactless distance sensor. It emits a probe signal 70, which is directed at a movable component formed by the drive chassis 10 of the drug delivery device 1. The probe signal 70 is reflected as a measurement signal 72 at a distal surface of the movable component 10 and received by the distance sensor 68.

A sensing access 75 is provided at the distal end of the drug delivery device 1. The sensing access 75 is transparent for both the probe signal 70 and the measurement signal 72. Furthermore, an interior space of the drug delivery device 1 between the holder 80 and the movable component 10 is also transparent for the probe signal 70 and the measurement signal 72. This interior space may be free from further components interacting with the probe signal 70 and/or the measurement signal 72. The sensing access 75 may comprise the interior space. The interior space may only comprise a part of the complete volume in between the holder 80 and the movable component 10 so that additional components that are not transparent to the signal 70, 72 may be placed in between the holder 80 and the movable component 10 away from the sensing access 75.

At the distal end, the sensing access 75 may be closed by a transparent cover that prevents ingress of materials, such as dust, water or the like, into the drug delivery device 1.

Like the fourth embodiment, also the second embodiment may comprise the sensing access 75 between the distance sensor 39 and the movable component 10.

Said Bluetooth add-on module 27 can be attached and detached from the distal end of the modified housing 3 at the holder 80 via demountable and/or releasable clip features 53. Other connections, such as bayonet locks, threads or the like, may be implemented with alternative embodiments. The add-on module 27 detects fitment to a drug delivery device 1 via the attachment switch 49 which interacts with a localized protrusion 55 on the distal end face of the housing 3. While this attachment switch 49 remains released, indicating that the add-on module 27 is not fitted or incorrectly fitted to a drug delivery device 1, the add-on module 27 will remain in a very low power state, conserving battery life.

When the attachment switch 49 is pressed, this indicates that the electronic module 27 has been fitted to a drug delivery device 1 and the electronic module 27 may be activated and enter a higher power state to check drug identification, switch or sensor operation sequence and conduct a system health check (e.g. confirm battery voltage levels). On fitment to the drug delivery device 1, the wake switch 37 is also pressed, via interaction with the distal surface of the plunger rod 11, which is accessible through an opening in the distal end of the housing 3. The height of the localized protrusion 55 on the distal end face of the housing 3 ensures that the attachment switch 49 is pressed before the wake switch 37 as the electronic module 27 is coupled to the drug delivery device 1 and released after the wake switch 37 on removal of the electronic module 27. The sequence of switch state change can be used to inform correct fitment of the electronic module 27 to a drug delivery device 1.

In addition, a defined sequence or combination of switch press events, and potentially the specific timing between two or more switch state change events, can be used to provide a means of user interaction with the electronic module 27, such as initiating pairing or manual synchronization of stored data with a smartphone. The user could be instructed to press one or more of the available switches in a specific sequence and/or for a certain duration to activate different module functions in the un-coupled state of the electronic module 27.

Similar to the wake switches 37 described above, both the attachment switch 49 and wake switch 37 in this variant are expected to be of a mechanical type to minimize quiescent current drain in storage.

Once the electronic module 27 has confirmed attachment to a drug delivery device 1, it monitors the state of the wake switch 37 for indication that dispense has commenced. The wake switch 37 and the dose completion detection (for example the time of flight sensor 39 targeting the plunger rod 11 or MEMS microphone and signal processing to identify the end of dose click) is functionally identical to the arrangement described above with respect to the second exemplary embodiment. Thus, the further function of fourth embodiment is not described again in detail.

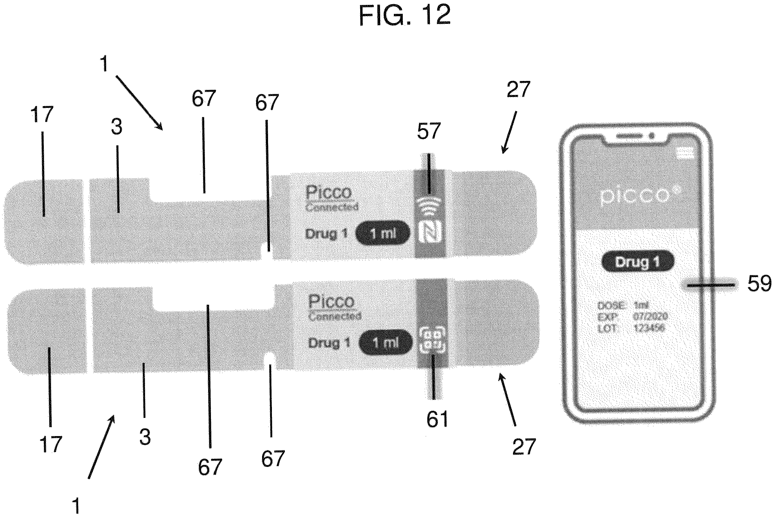

A further key aspect of a connected drug delivery system 1 is the ability to confirm the type and expiry of the drug to be delivered, providing the opportunity to warn or coach the user before an error is made. In particular, given an add-on electronic module 27 there is the potential to fit the electronic module 27 to drug delivery devices 1 containing different drugs. For such an add-on module 27, three possible solutions to address this concern are presented in the following with reference to FIGS. 12 to 14.

As illustrated in FIG. 12, according to a first exemplary configuration, an NFC tag 57 is embedded in a label on the housing 3 of the drug delivery device 1. This NFC tag 57 contains information about the drug stored within the drug delivery device 1. The NFC tag 57 is then read independently by a smartphone 59, in particular prior to the administering process. This data can be stored in the smartphone 59 and then potentially can be combined with dose delivery data provided by the add-on module 27 after completion of drug administering process. Alternatively, a barcode or QR code 61 is printed onto the label to be read by the camera of the smartphone 59. Thus, a user can be informed about specific information of the drug delivery device 1 prior to use to guide the following administering process.

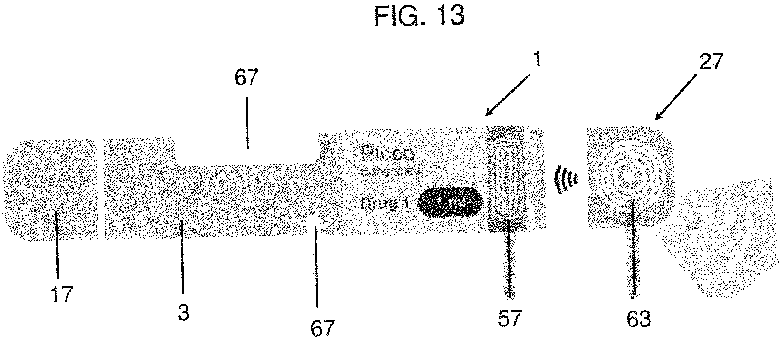

Referring to FIG. 13, an NFC reader 63 can be incorporated into the add-on electronic module 27 to automatically read the NFC tag 57 of the drug delivery device 1 as described above. This avoids the need for additional user steps of independently reading the NFC tag 57 with the smartphone 59 and also more reliably couples the drug information with the dose administering process. When the attachment switch 59 is pressed on fitment of the add-on module 27 to the drug delivery device 1, the built-in NFC reader 63 is activated to detect the drug information. This provides the opportunity to warn a user if the incorrect combination of add-on module 27 and drug delivery device 1 have been connected, either through further Bluetooth communication to the smartphone 59, or via the notification LED's of the electronic module 27 itself. If the drug information shows the medication is acceptable then positive confirmation may also be provided. Following the completion of the drug administering process, the drug information previously read by the add-on electronic module 27 may be combined with any dose event data and this information may be transmitted for example via Bluetooth to the smartphone 59 as a single record.

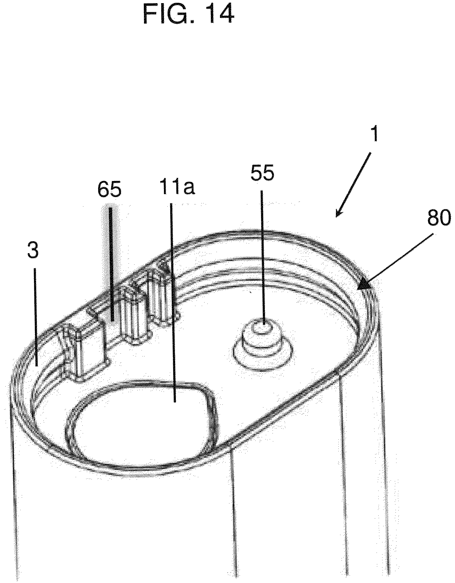

Finally, also mechanical coding features 65 can be incorporated into the interface between the add-on electronic module 27 and the housing 3 of the drug delivery device 1 to lock or code certain modules 27 so these can only be assembled to specific drug delivery devices 1. An example for such mechanical coding features 65 is illustrated in FIG. 14. For such an arrangement it is considered unlikely that sufficient resolution in the coding system could be realized to achieve unique expiry or batch identification, but drug specific coding is considered feasible.

Referring again to the wireless communication of the electronic module 27, the usage of Bluetooth protocol for communication permits two-way communication and therefore enables the smartphone 59 to confirm successful receipt of the data to the add-on electronic module 27, preventing unnecessary retransmission of data which would reduce battery life.

With the described add-on electronic module 27, following use with one first drug delivery device 1, the patient removes the add-on electronic module 27 from the depleted drug delivery device 1 and either immediately attaches it to a new drug delivery device 1 or stores it independently awaiting fitment to another drug delivery device 1, with which the administering process can be repeated.

It is pointed to the fact that the above described drug delivery devices 1 are single use fixed dose drug delivery devices. Accordingly, only one single dose of a preset amount depending on the internal structural configuration of the drug delivery device 1 can be dispensed with these drug delivery devices 1.

However, the above described drug administering process surveillance is not restricted to such single use fixed dose drug delivery devices 1. If desired, the above described functionalities can also be combined with multiple use fixed dose drug delivery devices, allowing the dispensing of multiple preset doses, single use variable dose drug delivery devices, allowing the dispensing of a single dose of a user-selectable amount, and multiple use variable dose drug delivery devices, allowing the dispensing of multiple doses of a user-selectable amount.

Finally, it is pointed to the finding that configuring the drug delivery device 1 and the electronic module 27 in such a manner that the external surface of the overall product in the connected state is flush seen along the longitudinal axis of the drug delivery device 1 without any steps or protrusions allows a highly improved handling of the drug delivery device 1 with the electronic module 27 by a user. Such a configuration is implemented in all the illustrated embodiments. The cutouts illustrated in FIGS. 12 and 13 depict only windows 67 within the housing 3 but no real recesses perpendicular to the longitudinal axis of the drug delivery devices 1.

The wake switches 37 of the foregoing embodiments form specific examples of wake sensors. With other embodiments, the wake sensors may be different from switches and/or different from mechanical and/or electromechanical switches. Likewise, the attachment switches 49 of the foregoing embodiments form specific examples of attachment sensors. With other embodiments, the attachment sensors may be different from switches and/or different from mechanical switches and/or different from electromechanical switches.

In all cases, sensors that are different from switches and/or different from mechanical switches and/or different from electromechanical switches may be sensors that are configured to detect a multitude of mutually differing measurement values, such as discrete or continuous measurement values, and that are configured to provide corresponding sensor signals. A sensor status, such as a binary sensor status, then may be determined by comparing the measurement value to a predetermined threshold. Such a sensor may form a specific embodiment of a switch that is different from a mechanical and/or electromechanical switch.

In general, the sensors like the wake switches 37 and the attachment switches 49 may be switches different from mechanical switches and/or different from electromechanical switches.

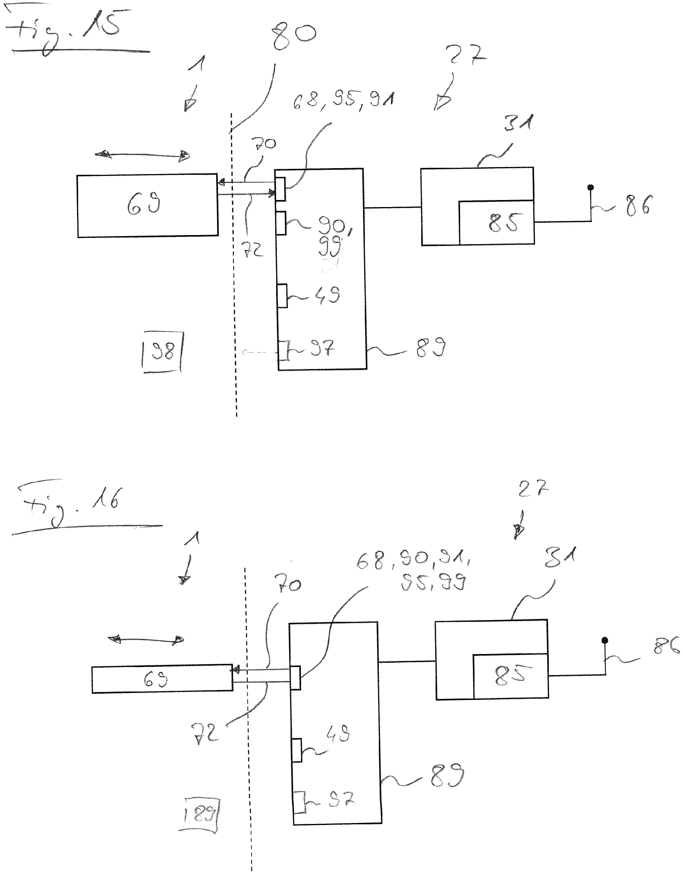

Fig. 15 schematically illustrates a set comprising an electronic module 27 and a drug delivery device 1 in accordance with the disclosure, when the electronic module 27 is connected to the drug delivery device 1 via a holder 80. The drug delivery device 1 may be a disposable autoinjector, but is not limited thereto. For example, the drug delivery device 1 and the electronic module 27 may be a respective drug delivery device 1 and electronic module 27 according to one of the first to fourth exemplary embodiments described above.

The electronic module 27 comprises sensing electronics 89 and a processing unit 31, which may be a micro-controller. The sensing electronics 89 is connected to the processing unit 31 to transfer signals that we present sensor states of the sensing electronics 89 to the processing unit 31. The processing unit 31 comprises a communication unit 85 that communicates with external devices via an antenna 86. The processing unit 31 and the communication unit 85 may be a combined system or component.

The sensing electronics 89 comprises a new pen sensor 90 configured to generate a signal indicating whether the drug delivery device 1 is new or has already been used. The new pen sensor 90 is triggered when the electronic module 27 is attached to a new drug delivery device 1. A designator 98 of the drug delivery device, such as an internal unique ID IC ("serial number IC") of the of the drug delivery device 1, can be read by the electronic module 27, in particular by the microcontroller 31, and can be stored in a memory (not shown) of the electronic module 27. The designator 98 may be read via an identification sensor 97 of the electronic module 27.

The processing unit 31 is configured to determine from a sensor signal of the new pen sensor 90 as a status of the drug delivery device 1 whether the drug delivery device 1 is new or has already been used.

The new pen sensor 90 is configured to sense a position, such as an axial position, of a movable component 69 of the drug delivery device 1. The movable component 69 takes up an initial position, for example with respect to the holder 80, when the drug delivery device 1 is new and moves away from the initial position upon a first use of the drug delivery device 1. Furthermore, the movable component 69 may never reach the initial position again during subsequent use of the drug delivery device 1.

With the fourth embodiment of the drug delivery device 1 shown in Fig. 11, the new pen sensor 90 may be exemplarily formed by the wake switch 37 and the movable component 69 may be formed by the drive chassis 10. A sensor status change of the new pen sensor 90 indicating the attachments to a new drug delivery device 1 then may be a status change of the wake switch 37 that occurs when the wake switch 37 is pressed.

Furthermore, the sensing electronics 89 comprises an end of dose sensor 95 that is configured to generate a signal indicating a completion of a drug delivery process. The processing unit 31 is configured to determine the completion of drug delivery as a status of the drug delivery device 1 based on the signal of the end of dose sensor 95.

With the fourth embodiment of the drug delivery device 1 shown in Fig. 11, the end of dose sensor 95 may be formed by the distance sensor 68. The processing unit 31 may determine the completion of drug delivery by comparing a distance measured by the distance sensor 68 with a predetermined end of dose distance. With the first embodiment of the drug delivery device 1, the end of dose sensor may be formed by the wake sensor 37 and/or the linear movement sensor formed by the photo-reflector sensor 29.

In addition, the sensing electronics 89 comprises a dispense sensor 91 that is configured to generate a signal indicating a progress of a drug delivery process as a status of the drug delivery device 1. The processing unit 31 is configured to determine and/or monitor the progress of drug delivery as a status of the drug delivery device 1 based on the signal of the dispense sensor 91.

With the fourth embodiment of the drug delivery device 1 shown in Fig. 11, the dispense sensor 91 may be formed by the distance sensor 68. The processing unit 31 may determine the progress of drug delivery from a distance measured by the distance sensor 68. The distance thereby is correlated, such as proportional, to the already dispensed amount of dose. With the first embodiment of the drug delivery device 1, the dispense sensor 91 may be formed by the linear movement sensor formed by the photo-reflector sensor 29.

The sensing electronics 89 also comprises an attachment sensor 49 that is configured to generate a signal indicating attachment of the electronic module 27 to the drug delivery device 1 as a status of the drug delivery device 1. The processing unit 31 is configured to determine attachment of the electronic model 27 as a status of the drug delivery device 1 based on the signal of the attachment sensor 49.

Furthermore, the sensing electronics 89 comprises an activation sensor 99 that is configured to generate a signal indicating an activation of the drug delivery device, such as a beginning of dose delivery as a status of the drug delivery device 1. The processing unit 31 is configured to determine the activation of the drug delivery device 1 as a status of the drug delivery device 1 based on the signal of the activation sensor 99.

With the fourth embodiment of the drug delivery device 1 shown in Fig. 11, the activation sensor 99 may be exemplarily formed by the wake switch 37. A sensor status change of the activation sensor 99 indicating the activation of the drug delivery device then may be a further status change of the wake switch 37 that occurs when the wake switch 37 is released. In particular, the further status change may be an inverse status change compared to the status change indicating that the electronic module 27 is attached to a new drug delivery device 1, which is evaluated when the wake switch 37 is used as the new pen sensor 90. In particular, the wake switch 37 may serve both as the new pen sensor 90 and the activation sensor 99.

With alternative embodiments of the drug delivery device 1 shown in Fig. 15, the new pen sensor 90 and/or the activation sensor 99 may also be formed by the distance sensor 68. The processing unit 31 and/or the distance sensor 68 may be configured to compare a distance measured by the distance sensor 68 to a predetermined new pen distance and to infer that the drug delivery device 1 is new when the distance equals the new pen distance. Additionally or alternatively, the processing unit 31 and/or the distance sensor 68 may be configured to determine the activation of the drug delivery device 1, such as a beginning of drug delivery, by determining that the measured distance starts to deviate from the new pen distance.

A further embodiment of the set shown in Fig. 15 that uses the distance sensor 68 both as the new pen sensor 90 and the activation sensor 99 is exemplarily shown in Fig. 16.

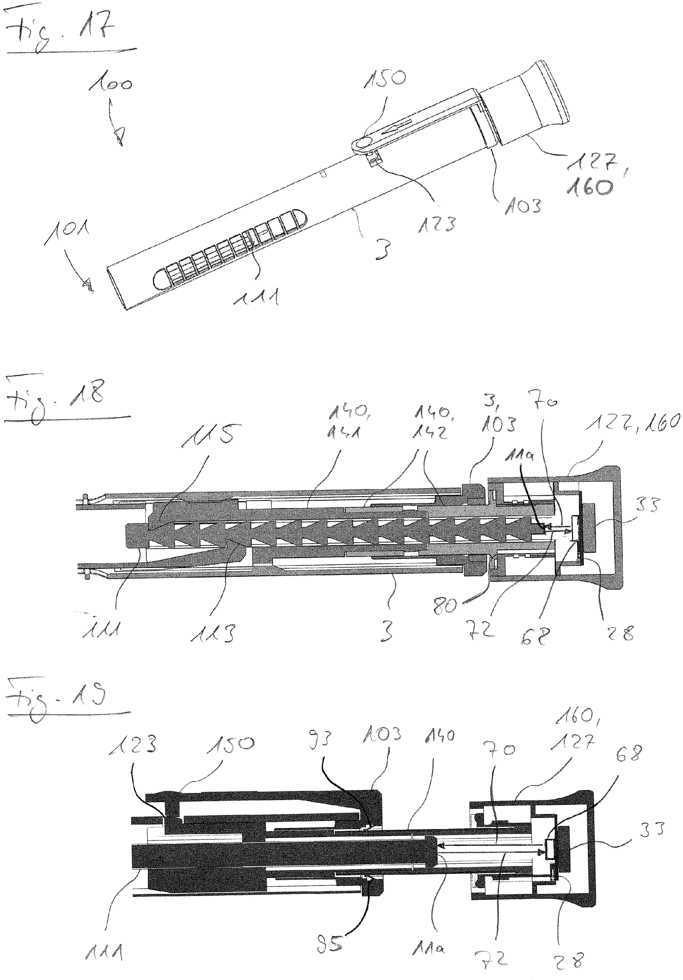

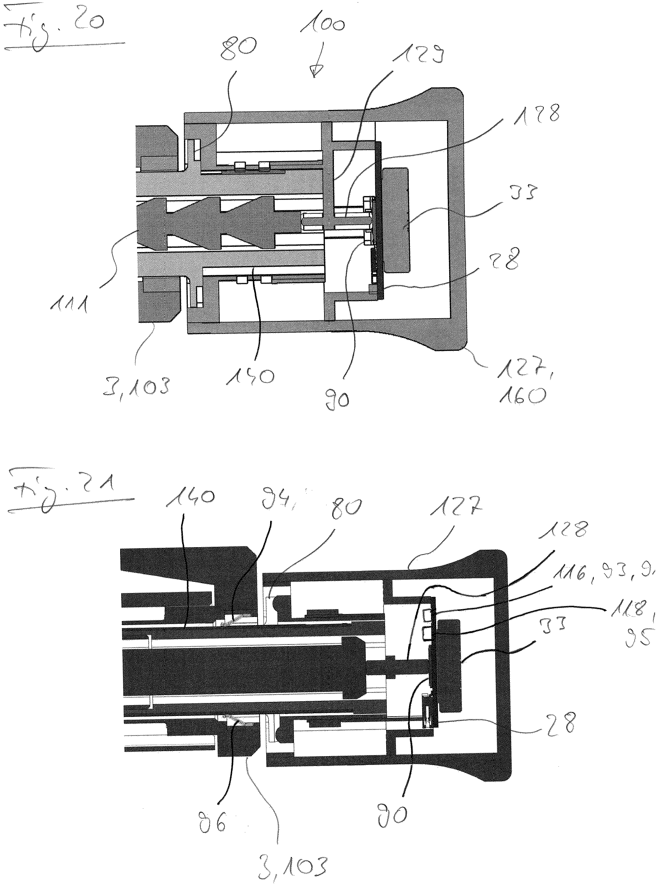

Fig. 17 show a perspective view of a fifth exemplary embodiment of a drug delivery device 100 and electronic module 127 according to the present disclosure, Fig. 19 shows a cross-sectional side view of the fifth embodiment with the drug delivery device 100 in a zero dose status and Fig. 20 shows a cross-sectional side view of the fifth embodiment with the drug delivery device in a set dose status. As far as no differences are disclosed, the fifth exemplary embodiment is configured as it is disclosed for the fourth exemplary embodiment and vice versa.

The drug delivery device 100 is a disposable multiple use fixed dose device. It allows for repeated injection of the same fixed dose of medicament from a medicament container (not shown) provided at a proximal needle end 101 of the drug delivery device 100. After injection of a last dose from the medicament container, the drug delivery device 100 is disposed of. In particular, the medicament container, such as a cartridge, may be fixedly and non-releasably held within the device 100.

The drug delivery device 100 has a plunger rod 111 held within a housing 3. The plunger rod 111 is configured to only move in the proximal direction with respect to the housing 3 and movement in the distal direction is prevented. The plunger rod 111 is coupled to the housing 3 via a unidirectional coupling 113 that is exemplarily configured as a ratchet between the piston rod 111 and the housing 3.

Furthermore, the drug delivery device 100 comprises a drive sleeve 140 that surrounds the piston rod 111 and that couples to the piston rod 111 to move it in the proximal direction during dose delivery. The drive sleeve 140 exemplarily comprises a proximal part 141 and a distal part 142 that are rigidly fixed to each other. With the exemplary embodiment shown, the drive sleeve 140 directly engages the piston rod 111. With other embodiments, the drive sleeve 140 may couple to the piston rod 111 via one or more intermediate components.

The drive sleeve 140 couples to the piston rod 111 via a drive lock 115 that forces the piston rod 111 to move axially in the proximal direction with the drive sleeve 140 during dose delivery. The drive lock 115 is further configured to allow axial movement of the drive sleeve 140 in the distal direction relative to the piston rod 111 during dose setting.

With the fifth exemplary embodiment, the drive lock 115 is formed by a further unidirectional coupling that blocks movement of the drive sleeve 140 relative to the piston rod 111 in the proximal direction and allows relative movement in the distal direction.

The drug delivery device 100 does not allow for dose correction. A set dose has to be delivered prior to setting a new dose.

The electronic module 127 is releasably attachable to the drug delivery device 100 via a holder 80 that is provided at the drive sleeve 140.

When the drug delivery device 100 is new and the electronic module 127 is attached, it has the status shown in Fig. 18. Before delivering a dose, the device 100 is activated and the dose is set by moving a dose dial 160 with respect to the housing 3. The dose dial 160 is exemplarily formed by the electronic module 127 attached to the device 100.

To set the dose and to activate the device 100, the dose dial 160 is moved in the distal direction. With the present embodiment, the dose dial 160 only performs a linear motion in the distal direction. Distal movement of the dose dial 160 also moves the drive sleeve 140 in the distal direction. The drive sleeve 140 is axially fixed with respect to the dose dial 160, in particular during dose setting.

The dose is set and the device 100 is activated when the movement of the dose dial 160 is stopped by a dose stop. The dose stop is exemplarily configured between the housing 3 and the drive sleeve 140. At the dose stop, the drive sleeve 140 engages with the housing 3 preventing further distal movement of the drive sleeve 140.

The dose dial 160 and the drive sleeve 140 move in the distal direction against a biasing force provided by a spring (not shown) that is provided between the housing 3 and the drive sleeve 140. Distal movement of the drive sleeve 140 and the dose dial 160 thereby tensions the spring.

The drug delivery device 100 is shown in Fig. 19 in the activated status with the dose set. The dose dial 160 and the drive sleeve 140 are held in a distal position corresponding to a set dose position or an activated position with respect to the housing 3. Thereby, a locking element 123 blocks proximal movement of the drive sleeve 140, the dose dial 160 and also the plunger rod 111, in particular against the biasing force of the spring.

The locking element 123 is exemplarily provided between the housing 3 and the drive sleeve 140. The drive sleeve 140 and the housing 3 are engaged with each other via the locking element 123. Thereby, the locking element 123 is formed at one of the housing 3 and the drive sleeve 140, namely at the drive sleeve 140, and the locking element 123 is engaged with the other one of the housing 3 and the drive sleeve 140, namely with the housing 3.

With the drug delivery device 100, the drive sleeve 140 forms a first component that is configured to perform a movement with respect to a second component formed by the housing 3 during the dose setting operation and the dose delivery operation. The locking element 123 thereby locks the first component 140 to the second component 3 prior to dose delivery.

The drug delivery device 100 further comprises a trigger 150 that is configured to perform an unlocking movement to displace the locking element 123 to initiate dose delivery. With the drug delivery device 100, the trigger 150 is formed by a clip provided at an outside surface of the housing 3.

After unlocking of the locking element 123, the drive sleeve 140 and the plunger rod 111 are driven in the proximal direction by the spring to deliver the set dose. Dose delivery is completed when the drive sleeve 140 and the dose dial 160 have again reached the proximal position shown in Fig. 18. Compared to the status shown in Fig. 18, the plunger rod 111 has been driven in the proximal direction during dose delivery so that it has been displaced from its initial position shown in Fig. 18.

The electronic module 27 comprises a distance sensor 68 configured to measure a distance from the distance sensor 68 to a surface 11a of the plunger rod 111 disposed in the drug delivery device 1. To this end, the distance sensor 68 emits a probe signal 70 towards a surface 11a of the plunger rod 111 and receives a measurement signal 72 from said surface 11a, the surface 11a being disposed on a distal end of the plunger rod 111 facing the distance sensor 68. Based on the measurement signal 72, the distance sensor 68 generates an electronic signal indicating a distance between the distance sensor 68 and the surface 11a of the plunger rod 111.

The electronic module 27 further comprises a processing unit (not shown) configured to receive the generated signal from the distance sensor 68 and to acquire, based on the received signal, information about at least one status of the drug delivery device 100. In particular, the distance is characteristic for a current or actual status of the drug delivery device 100, such as a state of use. For example, the distance indicates at least one of whether the drug delivery device 100 is new or has already been used, whether the electronic module 127 is properly mounted, whether a dose has been set or whether the set dose has been entirely delivered.

The distance sensor 68 may form a new pen sensor. The distance measured by the distance sensor 68 may be compared to a new pen distance by the processing unit and the status of the drug delivery device 100 may be determined to be new if the measured distance equals the new pen distance. The new pen distance may be the shortest distance between the plunger rod 111 and the distance sensor 68 during the use of the drug delivery device 100.

The distance sensor 68 may additionally or alternatively form an activation sensor that provides a signal that indicates that the drug delivery device 100 has been activated, for example by setting the dose. The signal may indicate that the dose setting has started or it may indicate that the dose setting has been completed. The distance sensor 68 may provide a signal indicating that the distance between the plunger rod 111 and the distance sensor 68 has increased from an initial distance in the zero dose position. The processing unit then may determine that dose setting has started. The signal also may indicate that the distance has increased by a dose setting distance. The dose setting distance equals the distance that the drive sleeve 140 travels from the initial position shown in Fig. 18 to the dose setting position shown in Fig. 19. The processing unit then may determine that dose setting has been completed.

The drug delivery device 100 additionally may have an end of dose sensor that delivers a signal indicating that a set dose has been completely delivered. The end of dose sensor may be configured to perform a status change upon the dose dial 160 and the drive sleeve 140 reaching and/or leaving their initial position with respect to the housing 3. For example, the end of dose sensor may be configured as a switch that is triggered when the dose dial 160 and the drive sleeve 140 are in the initial position. The end of dose sensor may, for example, be provided at a proximal end of the electronic module 127. When being configured as a switch, it may be activated by a component rigidly connected to the housing 3.

With alternative embodiments, the end of dose sensor may also be configured as a distance sensor that is provided at the electronic module 127 and that generates a signal indicating a distance between the distance sensor and the housing 3. End of dose delivery then may be detected by the processing unit by the measured distance being equal to an end of dose distance between the housing 3 and the distance sensor in the initial state shown in Fig. 18.

Figs. 20 and 21 show a sixth embodiment of an electronic module 127 for a drug delivery device according to the present disclosure. As far as no differences are disclosed, the sixth embodiment is configured as it is disclosed for the fifth embodiment shown in Figures 17 to 19 and vice versa. In particular, the sixth embodiment of the electronic module 127 is configured to be attached to the drug delivery device 100.

The electronic module 127 comprises a new pen sensor 90. The new pen sensor 90 is provided at a proximal side of the electronic module 127, namely at a proximal side of the PCBA 28. The new pen sensor 90 is configured as an electromechanical switch. It is actuated by the plunger rod 111 in its distal initial position. Exemplarily, the new pen sensor 90 is actuated by an intermediate component configured by a rod. The intermediate component is connected to a housing of the electronic module 127 by a flexible connection 125.

Upon moving the dose dial 160 in the distal direction to set the first dose, the new pen sensor 90 performs a status change, namely a binary switch status change. Since the plunger rod 111 moves proximally together with the dose dial 160 during dose delivery, the new pen sensor 90 is not actuated again during subsequent use of the device 100.

As can be seen from Fig. 21, the electronic module 127 comprises a first sensor 116 and a second sensor 118. The first sensor 116 and the second sensor 118 are configured to detect binary switching events of switches connected with the sensors 116, 118. The switches are electromechanical switches. They are configured to perform binary switch status changes when being switched. A first status change changes the status of the switches from a first state to a second state and a second status change changes the status from the second state back to the first state. One of the states is a closed state and the other one of the states is an open state.

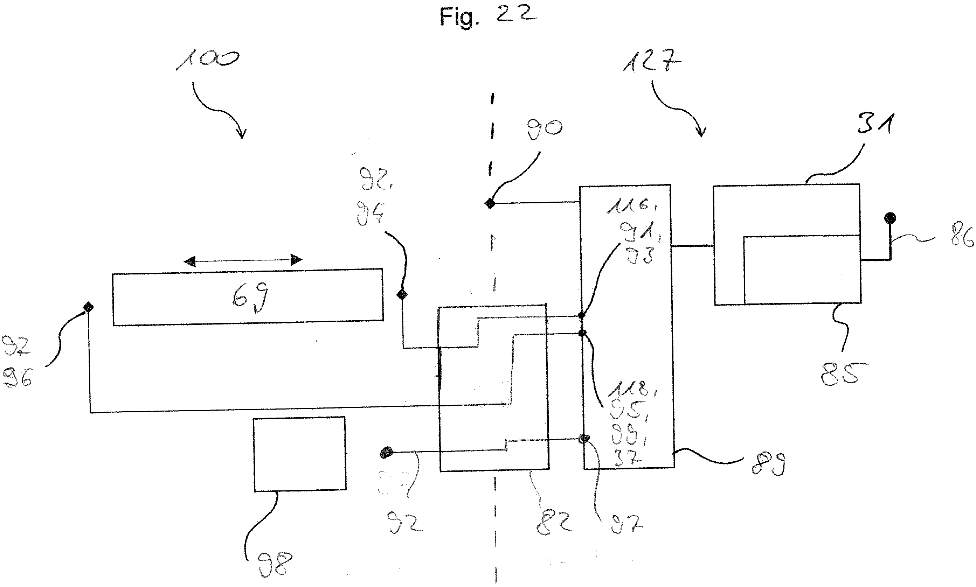

Fig. 22 shows a schematic diagram of the sixth embodiment of the electronic module 127 attached to the drug delivery device 100.

The first sensor 116 and the second sensor 118 are configured to detect the switching events based on the position of at least one movable component 69 of the drug delivery device 100 with respect to the housing 3. The first sensor 116 detects a switching event when the movable component 69 reaches a set dose position in which the dose has been set. Furthermore, the first sensor 116 again detects a switching event when the movable component 69 leaves the set dose position, for example upon dose delivery. The second sensor 118 detects a switching event when the movable component 69 reaches an end of dose position in which the dose has been completely delivered. The end of dose position also forms the initial position prior to dose setting. Furthermore, the second sensor 118 again detects a switching event when the movable component 69 leaves the end of dose position or initial position, for example upon setting a new dose.

With the first drug delivery device 100, the at least movable component 69 is formed by the dose dial 160 and/or the drive sleeve 140. The set dose position corresponds to the distal position of said movable components 69 shown in Fig. 19 and the dose delivery position corresponds to the proximal position of said movable components 69 shown in Fig. 18.

The second sensor 118 is a wake sensor 37. It detects the first status change from the first state to the second state when dose setting starts. The movable component 69 thereby leaves its initial position at the beginning of dose setting. Upon the first status change being detected by the second sensor 118, the processing unit 31 awakes from a sleep state and stores corresponding data in the memory of the electronic module 127.

The first sensor 116 is a dose setting sensor 93. It detects setting of a dose with the drug delivery device 100 as a switching event. The first sensor 116 detects the first status change from the first state to the second state when the dose has been set with the drug delivery device 100. The movable component 69 thereby reaches its dose setting position. Upon the first status change being detected by the first sensor 116, the processing unit 31 determines that a dose has been set and stores corresponding data in the memory of the electronic module 127. This also corresponds to an activation of the drug delivery device 100. The first sensor 116 therefore also forms an activation sensor 99.

Furthermore, the first sensor 116 is a dispense sensor 91. It detects the start of dose delivery. The first sensor 116 detects the second status change from the second state to the first state of the connected switch when dose delivery starts. The movable component 69 thereby leaves its set dose position. Upon the second status change being detected by the first sensor 116, the processing unit 31 determines that dose delivery has started and stores corresponding data in the memory of the electronic module 127.

The second sensor 118 is also an end of dose sensor 95. It is configured to detect a status change when the set dose has been completely delivered by the drug delivery device 100. The second sensor 118 detects the second status change from the second state to the first state when dose delivery has been completed. The movable component 69 thereby reaches its initial position. Upon the second status change being detected by the second sensor 118, the processing unit 31 determines that the end of dose delivery has occurred and stores corresponding data in the memory of the electronic module 127.

With alternative embodiments, the first sensor 116 may form the wake sensor. The processing unit 31 then may awake when the first sensor 116 detects the first status change also indicating the setting of the dose.

The electronic module 127 stores the data representing the dosing sequence with date and time record in the memory. As new data is available in the memory of the electronic module 127, the data is transmitted via the communication unit 85, for example via Bluetooth Low Energy (BLE), to an external device, such as the patient's smartphone. With alternative implementations of all communication units 85 according to the present disclosure, the data may also be transmitted using a publisher/subscriber protocol or the like. For example, an Internet-of-Things (loT) protocol may be used.

The electronic module 127 furthermore comprises an identification sensor 97, that is configured to detect a designator 98 on the drug delivery device 100. The designator 98 may be, for example, an internal unique ID integrated circuit (IC), an RFID tag, a barcode, an optical or conductive printing or the like. The data indicating the detected designator 98 is stored in the memory by the processing unit 31.

As can be seen from Fig. 22, the first and second sensor 116, 118 are connected to an electric sense circuit 92 provided at the drug delivery device 100 via an electrical connector 82. Furthermore, also the identification sensor 97 is connected to the sense circuit 92 to detect the designator 98. With alternative embodiments, the identification sensor 97 may also directly and/or contactless detect the designator 98 without any connection to the sense circuit 92.

The sense circuit 92 may comprise printed conductive structures located on the components of the drug delivery device 100, such as the drive sleeve 140. With alternative embodiments, the sense circuit 92 may comprise printed circuit boards, such as flexible printed circuit boards, that are fixed to said components or integrated molded structures of said components and that carry the conductors of the sense circuit 92.

The sense circuit 92 comprises first sensing conductors 94 that form the electromechanical switch connected to the first sensor 116 and second sensing conductors 96 that form the electromechanical switch connected to the second sensor 118.

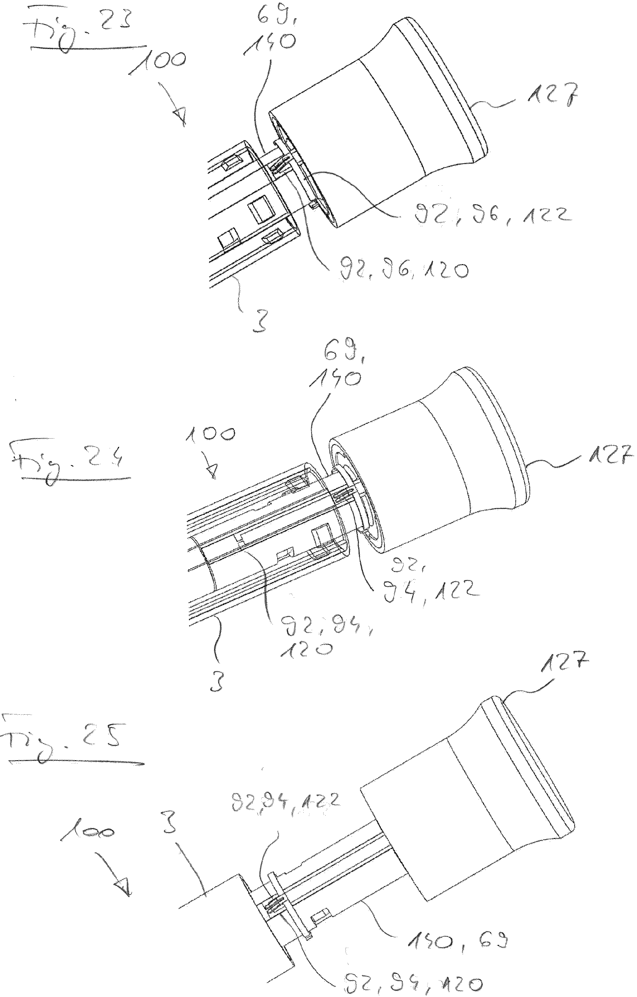

Fig. 23 shows the sense circuit 92 with the drug delivery device 100 in the initial state corresponding to the end of dose state in a first perspective view and Fig. 24 shows the sense circuit 92 with the drug delivery device 100 in the initial state corresponding to the end of dose state in a second perspective view. Fig. 25 shows the sense circuit 92 with the drug delivery device 100 in the activated state with a dose set in a perspective view.

The first and second sensing conductors 94, 96 each comprise circuit lines 120 fixed to the drive sleeve 140, whereby the circuit lines 120 are provided on a flexible printed circuit board fixed to the outer surface of the drive sleeve 140. In addition, the first and second sensing conductors 94, 96 each comprise a contact 122. The contacts 122 are fixed to the housing 3, which is shown transparent in Figures 23 and 24. The contacts 122 are thereby held within a ring-shaped holder fixed to the distal end of the housing 3, whereby the holder is not shown in Figures 23 to 25 for better visibility.

Each contract 122 is configured as a metal member and comprises two flexible elements that extend towards the drive sleeve 140 and that are configured to slide on the surface carrying the circuit lines 120 to conductively contact the circuit lines 120 depending on the relative position between the drive sleeve 140 in the housing 3. Upon contact, each contact 122 closes an electrical circuit formed by the respective circuit lines 120.

As can be seen from Fig. 23, the circuit lines 120 of the second sensing conductors 96 are contacted by the respective contact 122 in the initial state so that the corresponding electrical circuit is closed. Furthermore, as can be seen from Fig. 23, which shows the drug delivery device 100 from the opposite side in the initial state, the circuit lines 120 of the first sensing conductors 96 are not contacted by the respective contact 122 in the initial state so that the corresponding electrical circuit is open.

As soon as the drive sleeve 140 leaves the initial position, the circuit lines 120 of the second sensing conductors 96 move out of contact with the respective contact 122 so that the electrical circuit of the second sensing conductors 96 is opened.

In the activated state with a dose set shown in Fig. 25, the circuit lines 120 of the first sensing conductors 94 are contacted by their respective contact 122 and the corresponding electrical circuit is closed. As soon as the drive sleeve 140 leaves the dose setting position, the first sensing conductors 94 move out of contact with the respective contact 122 so that the electrical circuit of the first sensing conductors 94 is opened.

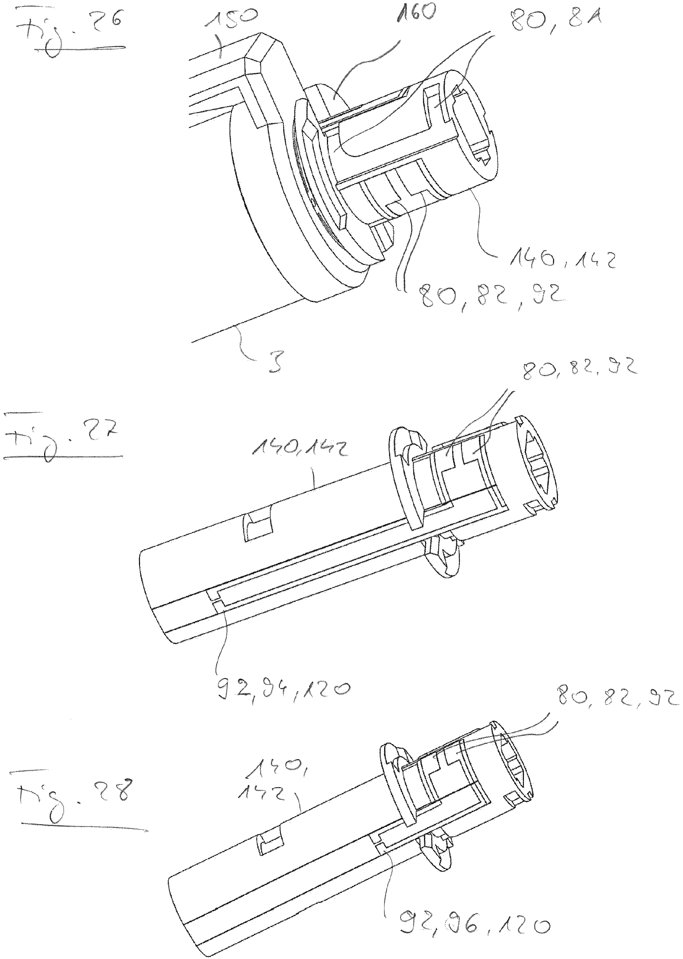

Figures 26 to 28 depict the holder 80 for mounting the electronic module 127 to the drug delivery device 100 of the sixth of embodiment. The holder 80 is provided at a distal end of the drive sleeve 140 and comprises a mechanical connector 81 that is configured as a bayonet lock and an electrical connector 82 that is formed by parts of the sensing conductors 94, 96 of the sense circuit 92 provided on the outer surface of the drive sleeve 140. The mechanical connector 81 is configured to connect to a complementary mechanical connector provided on an inside surface of the electronic module 127.

The electrical connector 82 is configured to connect to a complementary electrical connector that is also provided on an inside surface of the electronic module 127.

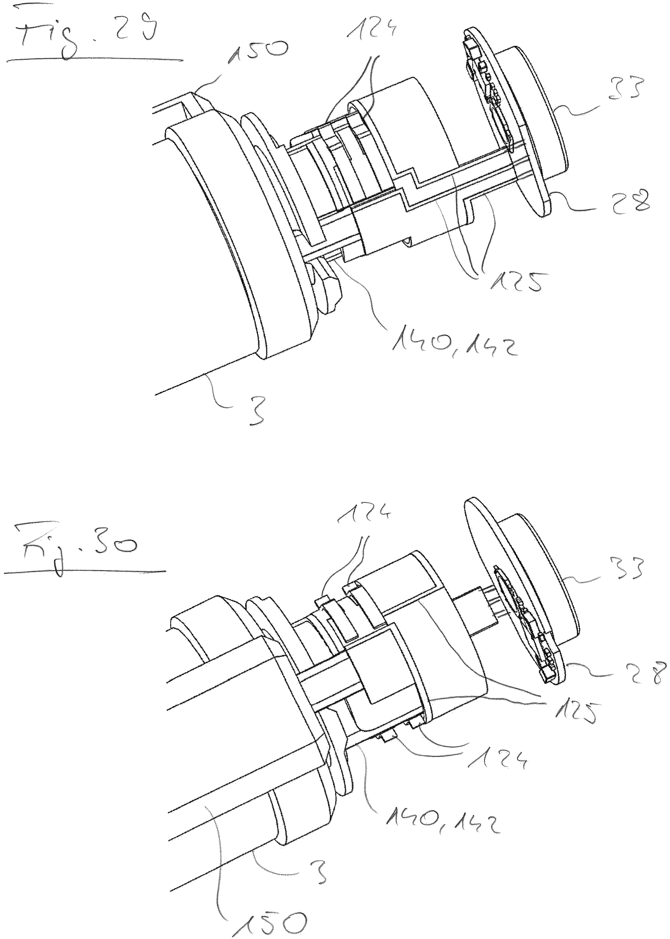

Figs. 29 and 30 depict the electrical connector of the electronic module 127, whereby an outer housing of the electronic module 127 is not shown for better visibility. The electrical connector comprises flexible contacts 124 that are configured to rest against the sensing conductors 94, 96 of the sense circuit 92 at the electrical connector 82 of the drug delivery device 100. Furthermore, the electrical connector of the electronic module 127 comprises circuit lines 125 that are provided on a flexible printed circuit board and that conductively connect the flexible contacts 124 with the printed circuit board 28 carrying the sensors 116, 118 and the processing unit 31.

With the embodiment shown in Figures 23 to 30, the electromechanical switches detected by the sensors 116, 118 each comprise the sensing conductors 94, 96 provided by the drug delivery device 100.

With alternative embodiments, the electromechanical switches read out by the sensors 116, 118 may also be located directly on the printed circuit board 28 and they may be actuated directly on the printed circuit board 28. The electronic module 127 and/or the drug delivery device 100 then may comprise mechanical actuators accessible at the distal end of the drug delivery device 100, for example protruding from the distal end of the drug delivery device 100, that trigger the electromechanical switches.

With the sixth embodiment, the electronic module 127 is a reusable part separate from the drug delivery device 100. Alternatively, the electronic module 127 may also be formed as an integral part of the drug delivery device 100.

The drug delivery devices 1, 100 according to the present disclosure may also comprise a lock that is configured to prevent a dose setting operation and/or a dose delivery operation in a locked state and to allow the dose setting operation and/or the dose delivery operation in an unlocked state. The lock thereby is in the locked state when the electronic module 27, 127 is not connected to the drug delivery device 1, 100 and the lock is configured to be in the unlocked state if the electronic module 27, 127 is connected to the drug delivery device. For example, the lock may be configured to transition from the locked state to the unlocked state upon the electronic module 27, 127 being connected to the drug delivery device 1, 100.

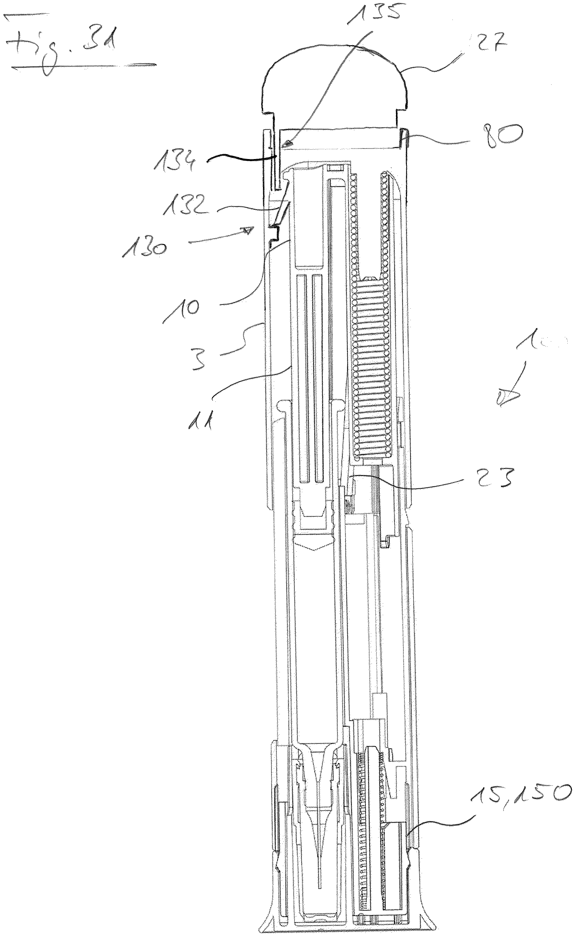

This is exemplarily shown in Fig. 31 for the drug delivery device 1 being in its initial state. The drug delivery device 1 comprises a first lock 130. The first lock 130 is configured to lock the first component formed by the drive chassis 10 to the second component formed by the housing 3 in its locked state. In the unlocked state of the first lock 130 the first component is released so that it is movable with respect to the second component, at least in principle.

The first lock 130 comprises a locking member 132 that locks the first component formed by the drive chassis 10 to the second component formed by the housing 3. The locking member 132 is a flexible member that is configured to be displaced to transfer the first lock 130 from the locked state to the unlocked state. Thereby, the locking member 132 is formed at one of the drive chassis 10 and the housing 3, exemplarily at the drive chassis 10, and engages with the other one of the drive chassis 10 and the housing, exemplarily with the housing 3. With other embodiments, the locking member 132 may also be formed at the housing 3 and engage with the drive chassis 10.

The electronic module 127 comprises an unlocking component 134 that is configured to displace the locking member 132 to transfer the first lock 130 from the locked state to the unlocked state. The unlocking component 134 thereby directly engages with the locking member 132.

The unlocking component 134 is provided at a proximal side of the electronic module 27 facing the drug delivery device 1 in the attached state. The unlocking component 134 thereby protrudes from the electronic module 27 towards the drug delivery device 1. It is received in an opening 135 of the drug delivery device 1. The opening 135 is provided at the distal end of the drug delivery device 1 and opens towards the electronic module 27. The unlocking component 134 is configured to actuate the locking member 130 through the opening 135.

When the first lock 130 is in the locked state, the first component formed by the drive chassis 10 will not move upon the trigger 150 formed by the needle guard 15 displacing the locking element 23 by the unlocking movement of the trigger 150. Reversing the movement of the trigger 150, for example by extending the trigger 150 back into its initial position, allows the locking element 23 to reengage with the housing 3.

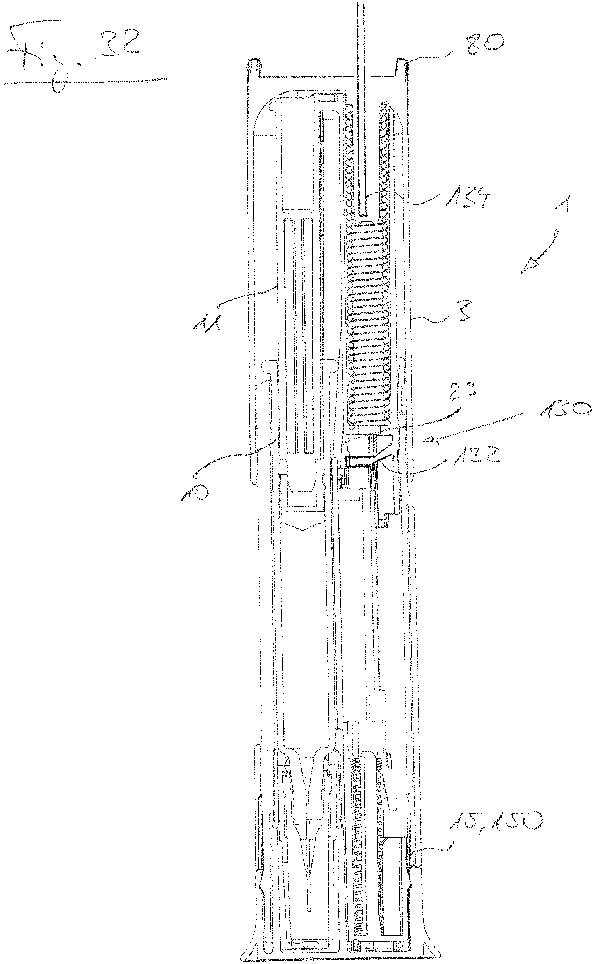

A second lock 130 for the drug delivery device 1 according to the present disclosure is shown in Fig. 32. The locking member 132 of the second lock 130 is configured to prevent movement of the locking element 23 in the locked state of the second lock 130. The locking member 132 thereby directly engages with the locking element 23 to prevent displacement of the locking element 23. Like with the first lock 130, the locking member 132 of the second lock 130 is displaced by an unlocking component 134 provided at the electronic module (not shown).

With alternative embodiments, movement of the locking element 23 may also be prevented by the locking member 132 of the second lock 130 restricting the movement of the trigger 150 formed by the needle guard 15. For example, the locking member 132 then may directly engage with the trigger 150.

With the drug delivery device 100, a lock corresponding to the first lock 130 shown in Fig. 31 may be provided between the drive sleeve 140 and the housing 3. Such a lock may lock the first component formed by the drive sleeve 140 to the second component formed by the housing 3 in the initial state and after completion of the dose delivery and detachment of the electronic module 127 from the drug delivery device 100. Such a lock prevents both dose setting and dose delivery being performed with the drug delivery device 100.

A lock corresponding to the second lock 130 shown in Fig. 32 may be configured to prevent movement of the locking element 123 of the drug delivery device 100. Thereby, the locking member of the lock may directly engage with the locking element 123 or it may restrict movement of the trigger 150 formed by the clip on the outside surface of the device.

In general, a drug delivery device that allows, like the drug delivery device 100, for dose setting, may comprise a component connecting the electronic module to the housing, whereby the component is configured to move relative to the housing during dose setting. Such a component, may, for example, be a dose dial, an actuation button or a sleeve connecting a dose dial and/or an actuation button to the drug delivery device. Said component then may form the first member that is locked to a second member to prevent dose setting and/or dose delivery.

In the following the basic features related to the present invention are listed with reference to specific aspects of the present invention:
A first aspect of the present invention refers to an electronic module (27) connectable to a drug delivery device (1) or a drug delivery device (1) with such an electronic module (27), wherein the electronic module (27) is configured to detect and to monitor a dose administering process with the drug delivery device (1) and/or parameters related thereto and to provide a user of the drug delivery device (1) with feedback on the conducted dose administering process and/or parameters related thereto.
A second aspect of the present invention refers to the electronic module (27) or drug delivery device (1) of the first aspect,
   wherein the drug delivery device (1) comprises: a housing (3); a cartridge or a syringe (5) filled with a drug to be dispensed and positioned within the housing (3); a plunger (7) positioned within the cartridge or syringe (5); a driver (9) positioned within the housing (3) and coupled to the plunger (7), wherein the driver (9) comprises a drive chassis (10) with a plunger rod (11) and a locked drive spring (13), wherein the locked drive spring (13) is acting between the housing (3) and the drive chassis (10) for generating the force necessary to move the plunger (7) for dispensing the drug; and a needle guard (15) provided axially movable within the housing (3) and configured to release the locked drive spring (13) of the driver (9) for dispensing the drug upon axial movement.
A third aspect of the present invention refers to the electronic module (27) or drug delivery device (1) of any one of the first and second aspect,
   wherein the electronic module (27) comprises a detection unit and a notification unit,
   wherein the detection unit is configured to monitor, i.e. surveil one or more moving parts of the drug delivery device (1) and to generate information about a administering process of the drug delivery device (1) or parameters related thereto based on an operating state of at least one component of the drug delivery device (1);
   wherein the notification unit is configured to generate and to output a notification signal including or indicating the generated information about the administering process or the parameters related thereto.
A fourth aspect of the present invention refers to the electronic module (27) or drug delivery device (1) of the third aspect,
   wherein the detection unit comprises a photo-reflector sensor (29) configured to monitor the movement of at least one component of the drug delivery device (1), in particular of a drive chassis (10) of the drug delivery device (1), during the administering process.
A fifth aspect of the present invention refers to the electronic module (27) or drug delivery device (1) of the fourth aspect,
   wherein the drive chassis (10) comprises at least one optical pattern (12) which is monitored by the photo-reflector sensor (29) for monitoring the dispensing movement of the plunger rod (11).
A sixth aspect of the present invention refers to the electronic module (27) or drug delivery device (1) of the third aspect,
   wherein the detection unit comprises a time of flight sensor (39) configured to monitor the movement of at least one component of the drug delivery device (1), in particular of a plunger rod (11) of the drug delivery device (1), during the administering process.
A seventh aspect of the present invention refers to the electronic module (27) or drug delivery device (1) of the sixth aspect,
   wherein the time of flight sensor (39) is an optical time of flight sensor and the plunger rod (11) comprises a reflective surface which is monitored by the optical time of flight sensor (39) for monitoring the dispensing movement of the plunger rod (11).
An eight aspect of the present invention refers to the electronic module (27) or drug delivery device (1) of the third aspect,
   wherein the detection unit comprises a directional microphone (39), in particular a micro-electromechanical system (MEMS) microphone, configured to detect a signal sound generated by at least one component of the drug delivery device (1), in particular of a drive chassis (10) of the drug delivery device (1), during the administering process.
A ninth aspect of the present invention refers to the electronic module (27) or drug delivery device (1) of the eight aspect,
   wherein the drive chassis (10) comprises at least one acoustic active section (25), in particular a clicker protrusion, generating an acoustic signal upon movement of the drive chassis (10) in the proximal direction, such that the directional microphone (39) can monitor the movement of the plunger rod (11) via the generated acoustic signal.
A tenth aspect of the present invention refers to the electronic module (27) or drug delivery device (1) of the third aspect,
   wherein the detection unit comprises at least one frangible circuit element (43) configured to be broken when at least one component of the drug delivery device (1), in particular a drive chassis (10) of the drug delivery device (1), reaches a specific position during the administering process, to monitor the administering process.
An eleventh aspect of the present invention refers to the electronic module (27) or drug delivery device (1) of any one of the third to tenth aspect,
   wherein the notification unit comprises an active wireless communicator (35) configured to transmit information actively wirelessly to a user device like a smart phone (59) or similar.
A twelfth aspect of the present invention refers to the electronic module (27) or drug delivery device (1) of the eleventh aspect,
   wherein the active wireless communication is based on the "cellular" communication technology SigFox.
A thirteenth aspect of the present invention refers to the electronic module (27) or drug delivery device (1) of the eleventh aspect,
   wherein the active wireless communication is based on the "cellular" communication technology NB-loT.
A fourteenth aspect of the present invention refers to the electronic module (27) or drug delivery device (1) of the eleventh aspect,
   wherein the active wireless communication is based on Bluetooth.
A fifteenth aspect of the present invention refers to the electronic module (27) or drug delivery device (1) of any one of the third to tenth aspect,
   wherein the notification unit comprises a passive wireless communicator (41) configured to transmit information passively wirelessly to a user device like a smartphone (59) or similar.
A sixteenth aspect of the present invention refers to the electronic module (27) or drug delivery device (1) of the fifteenth aspect,
   wherein the passive wireless communication is based on NFC (near-field communication), in particular with an RFID chip (45) and an antenna (47).
A seventeenth aspect of the present invention refers to the electronic module (27) or drug delivery device (1) of any one of the third to sixteenth aspect,
   wherein the notification unit comprises an optical notifier configured to directly display information to a user of the drug delivery device (1).
An eighteenth aspect of the present invention refers to the electronic module (27) or drug delivery device (1) of the seventeenth aspect,
   wherein the optical notifier includes at least one, in particular several, notification LED.
A nineteenth aspect of the present invention refers to the electronic module (27) or drug delivery device (1) of any one of the third to fourteenth, seventeenth and eighteenth aspect,
   wherein the electronic module (27) comprises a micro-controller (31) and a power supply (33),
   wherein the micro-controller (31) is configured to receive the information about the administering process from the detection unit, to process the receive information and to control the operation of the notification unit to output a user feedback based on the processed information.
A twentieth aspect of the present invention refers to the electronic module (27) or drug delivery device (1) of any one of the third to fourteenth and seventeenth to nineteenth aspect,
   wherein the electronic module (27) comprises a, in particular mechanical, wake switch (37),
   wherein the wake switch (37) is coupled to the micro-controller (31) and configured to switch the operation state of the micro-controller (31) between an ON state and an OFF state.
A twenty-first aspect of the present invention refers to the electronic module (27) or drug delivery device (1) of the twentieth aspect,
   wherein the wake switch (37) is configured not to be operated by a user of the drug delivery device (1) but automatically during the usage of the drug delivery device (1), in particular either by the needle guard (15) or the drive chassis (10) with the plunger rod (11).
A twenty-second aspect of the present invention refers to the electronic module (27) or drug delivery device (1) of any one of the preceding aspects,
   wherein the electronic module (27) is provided as integral component of the drug delivery device (1) non-releasably coupled to the components of the drug delivery device (1).
A twenty-third aspect of the present invention refers to the electronic module (27) or drug delivery device (1) of the twenty-second aspect,
   wherein the components of the electronic module (27) are positioned within a/the housing (3) of the drug delivery device (1).
A twenty-fourth aspect of the present invention refers to the electronic module (27) or drug delivery device (1) of the twenty-second or twenty-third aspect,
   wherein the electronic module (27) is positioned side by side with the components of the drug delivery device (1).
A twenty-fifth aspect of the present invention refers to The electronic module (27) or drug delivery device (1) of any one of the first to twenty-first aspect,
   wherein the electronic module (27) is provided as separate add-on module configured to be coupled releasably to the drug delivery device (1).
A twenty-sixth aspect of the present invention refers to the electronic module (27) or drug delivery device (1) of the twenty-fifth aspect,
   wherein the electronic module (27) comprises a sealing membrane (51) positioned between the electronic module (27) and the drug delivery device (1) in a state in which the electronic module (27) is coupled to the drug delivery device (1).
A twenty-seventh aspect of the present invention refers to the electronic module (27) or drug delivery device (1) of the twenty-sixth aspect,
   wherein the sealing membrane (51) is made of material that is transparent for specific light, in particular IR light, such that an optical time of flight sensor (39) can be used for monitoring the administering process through the sealing membrane (51).
A twenty-eighth aspect of the present invention refers to the electronic module (27) or drug delivery device (1) of any one of the twenty-fifth to twenty-seventh aspect,
   wherein the electronic module (27) comprises an attachment switch (49) configured to detect the attachment of the electronic module (27) to the drug delivery device (1).
A twenty-ninth aspect of the present invention refers to the electronic module (27) or drug delivery device (1) of any one of the preceding twenty-fifth to twenty-eighth aspect,
   wherein the drug delivery device (1) is provided with an NFC tag (57) containing information about the drug stored within the drug delivery device (1).
A thirtieth aspect of the present invention refers to the electronic module (27) or drug delivery device (1) of the twenty-ninth aspect with the features any one of the twenty-fifth to twenty-eighth aspect,
   wherein the electronic module (27) comprises an NFC reader (63) configured to automatically read the information stored within the NFC tag (57).
A thirty-first aspect of the present invention refers to the electronic module (27) or drug delivery device (1) of any one of the preceding aspects,
   wherein at least one component of the drug delivery device (1) comprises a barcode or QR code (61) containing information about the drug within the drug delivery device (1) and configured to be read by a user device like a smartphone (59).
A thirty-second aspect of the present invention refers to the electronic module (27) or drug delivery device (1) of any one of the preceding aspects with the features of any one of the twenty-firth to twenty-eighth and thirtieth aspect,
   wherein the electronic module (27) comprises a mechanical coding feature (65) only allowing the attachment of the electronic module (27) to drug delivery devices (1) provided with a matching mechanical coding feature.
A thirty-third aspect of the present invention refers to the electronic module (27) or drug delivery device (1) of any one of the preceding third to twenty-third and twenty-firth to thirty-second aspect not having the further features of the twenty-fourth aspect,
   wherein the electronic module (27) is configured to be or is positioned at a distal end of the drug delivery device (1) axially behind the components of the drug delivery device (1).
A thirty-fourth aspect of the present invention refers to the electronic module (27) or drug delivery device (1) of any one of the preceding aspects,
   wherein the drug delivery device (1) is a single use fixed dose drug delivery device.
A thirty-fifth aspect of the present invention refers to the electronic module (27) or drug delivery device (1) of any one of the preceding first to thirty-third aspect,
   wherein the drug delivery device (1) is a single use variable dose drug delivery device.
A thirty-sixth aspect of the present invention refers to the electronic module (27) or drug delivery device (1) of any one of the preceding first to thirty-third aspect,
   wherein the drug delivery device (1) is a multiple use fixed dose drug delivery device.
A thirty-seventh aspect of the present invention refers to the electronic module (27) or drug delivery device (1) of any one of the preceding first to thirty-third aspect,
   wherein the drug delivery device (1) is a multiple use variable dose drug delivery device.
A thirty-eighth aspect of the present invention refers to the electronic module (27) or drug delivery device (1) of any one of the preceding aspects,
   wherein the electronic module (27) is configured to detect and to monitor the dose administering process with the drug delivery device (1) and/or parameters related thereto based on a movement status of one or more moving parts of the drug delivery device (1) or based on a position of one or more moving parts of the drug delivery device (1).
A thirty-ninth aspect of the present invention refers to the electronic module (27) or drug delivery device (1) of any one of the preceding first to thirty-seventh aspect,
   wherein the electronic module (27) is configured to detect and to monitor the dose administering process with the drug delivery device (1) and/or parameters related thereto based on a movement status and based on a position of one or more moving parts of the drug delivery device (1).
A fortieth aspect of the present invention refers to the electronic module (27) or drug delivery device (1) of the thirty-ninth aspect,
   wherein the electronic module (27) is configured to detect and to monitor the dose administering process with the drug delivery device (1) and/or parameters related thereto based on the movement status and the position of one single moving part, in particular a drive chassis (10), of the drug delivery device (1).
A forty-first aspect of the present invention refers to the electronic module (27) or drug delivery device (1) of any one of the preceding aspects,
   wherein the micro-controller waits a specific period of time before finally determining the end of the dose administering process after no further change in anyone of the monitored parameters is changing any more.
A forty-second aspect of the present invention refers to the electronic module (27) or drug delivery device (1) of the forty-first aspect,
   wherein the specific period of time is 1, 2, 3, 5 or 10 seconds.
A forty-third aspect of the present invention refers to the electronic module (27) or drug delivery device (1) of any one of the preceding aspects,
   wherein an outer contour of the drug delivery device (1) together with the electronic module (27) is flush seen along the longitudinal axis of the drug delivery device (1).
A forty-fourth aspect of the present invention refers to the electronic module (27) or drug delivery device (1) of any one of the preceding aspects,
   wherein the electronic module (27) is configured to monitor the movement of a part of the drive chassis (10) during the administering process.

Further aspects of the present disclosure relate to the following embodiments:
1. An electronic module (27, 127) for a drug delivery device (1, 100), wherein the electronic module (27, 127) comprises
   a distance sensor (39) configured to generate a signal indicating a distance between the distance sensor (39) and at least one component (10, 111, 69) of the drug delivery device (1, 100) connected to the electronic module (27, 127), and
   a processing unit (31) configured to receive the generated signal from the distance sensor (39) and to acquire, based on the received signal, information about at least one status of the drug delivery device (1, 100).
2. The electronic module (27, 127) of embodiment 1,
   wherein the distance sensor (39) is different from a time-of-flight sensor and/or from an ultrasonic sensor and/or from a photo reflector sensor.
3. The electronic module (27, 127) of any one of the preceding embodiments, wherein the distance sensor (39) is based on at least one of a phase-shift measurement, a triangulation measurement and an intensity modulation measurement.
4. The electronic module (27, 127) of any one of the preceding embodiments, wherein the distance sensor (39) is one of an ultrasonic sensor, a RADAR sensor, a LIDAR sensor and/or an IR sensor.
5. The electronic module (27, 127) of any one of the preceding embodiments, wherein the distance sensor (39) is positioned on a proximal side of the electronic module (27, 127) facing the attached drug delivery device (1, 100).
6. The electronic module (27, 127) of any one of the preceding embodiments, wherein the distance sensor (39) is a contactless distance sensor (39) configured to emit a probe signal (70) towards the component (10, 111, 69) of the drug delivery device (1, 100) and to receive a reflection of the probe signal (70) as a measurement signal (72) from the drug delivery device (1, 100).
7. The electronic module (27, 127) of any one of the preceding embodiments, wherein the contactless distance sensor (39) is configured to emit the probe signal (70) and/or to receive the measurement signal (72) in a direction essentially parallel, such as parallel, to a longitudinal axis of the drug delivery device (1, 100).
8. The electronic module (27, 127) of any one of the preceding embodiments, wherein processing unit (31) is configured to acquire the information about the at least one status of the drug delivery device (1, 100) as information about a status indicating dose dispensing performed on the drug delivery device (1, 100),
   for example by comparing a distance derived on the basis of the signal received from the distance sensor (39) with a dispense distance,
   wherein, for example, the dispense distance is predetermined distance stored in a memory of the electronic module (27, 127).
9. The electronic module (27, 127) of embodiment 8,
   wherein the status indicating the dose dispensing indicates whether dose dispensing has started.
10. The electronic module (27, 127) of at least one of embodiments 8 and 9,
   wherein the status indicating the dose dispensing indicates whether dose dispensing has been completed.
11. The electronic module (27, 127) of at least one of embodiments 8 to 10,
   wherein the status indicating the dose dispensing indicates at least one of an amount of a dose having been dispensed during the dose dispensing, a date of the dose dispensing and a time of the dose dispensing.
12. The electronic module (27, 127) of any one of the preceding embodiments,
   wherein the processing unit (31) is configured to acquire the information about the at least one status of the drug delivery device (1, 100) as information about whether the drug delivery device (1, 100) is new or has already been used, for example by comparing a distance derived on the basis of the signal received from the distance sensor (39) with a new pen distance, wherein, for example, the new pen distance is predetermined distance stored in a memory of the electronic module (27, 127).
13. The electronic module (27, 127) of any one of the preceding embodiments, wherein the processing unit (31) is configured to acquire the information about the at least one status of the drug delivery device (1, 100) as information about the process of mounting the electronic module (27, 127) on the drug delivery device (1, 100), for example, by comparing a distance derived on the basis of the signal received from the distance sensor (39) with a mounting distance, wherein, for example, the mounting distance is a predetermined distance stored in a memory of the electronic module (27, 127).
14. The electronic module (27, 127) of embodiment 13,
   wherein the information about the process of mounting the electronic module (27, 127) on the drug delivery device (1, 100) comprises at least one of information about whether the mounting has been successful, a date of the successful mounting, a time of the successful mounting and a number of mounting attempts.
15. The electronic module (27, 127) of any one of the preceding embodiments,
   wherein the processing unit (31) is configured to acquire the information about the at least one status of the drug delivery device (1, 100) as information about the dose dialing performed on the drug delivery device (1, 100),
   for example, by comparing a distance derived on the basis of the signal received from the distance sensor (39) with a dialing distance, wherein, for example, the dialing distance is a predetermined distance stored in a memory of the electronic module (27, 127).
16. The electronic module (27, 127) of embodiment 15,
   wherein the information about the dose dialing performed on the drug delivery device (1, 100) comprises at least one of an amount of a dose having been set during the dose dialing, a date of the dose dialing and a time of the dose dialing.
17. The electronic module (27, 127) any one of the preceding embodiments,
   wherein the processing unit (31) is configured to store the information about the at least one status of the drug delivery device (1, 100) in a memory of the electronic module (27, 127), wherein, for example, the electronic module (27, 127) further comprises a communication unit configured to communicate said information to an external device (59).
18. The electronic module (27, 127) of any one of the preceding embodiments,
   wherein the electronic module (27, 127) further comprises a wake switch (37) configured to be triggered by a mechanical and/or electrical wake component (3, 10, 111, 69) of the drug delivery device (1, 100) and configured to generate a wake signal upon being triggered, wherein the electronic module (27, 127), in particular the processing unit (31), is configured to switch from a sleep mode to a wake mode in response to receiving the wake signal from the wake switch (37).
19. The electronic module (27, 127) of any one of the preceding embodiments,
   wherein the electronic module (27, 127) comprises a mechanical and/or electrical unlocking component configured to unlock a mechanical and/or electrical locking component of the drug delivery device (1, 100) that blocks dose dialing and/or dose dispensing.
20. A drug delivery device (1, 100) comprising a holder (80) for holding the electronic module (27, 127) according to any one of the preceding embodiments.
21. The drug delivery device (1, 100) of embodiment 20,
   wherein the component (10, 111, 69) of the drug delivery device (1, 100) and the holder (80) are configured to perform at least a linear movement relative to each other upon the dose dialing and/or dose dispensing.
22. The drug delivery device (1, 100) of any one of embodiments 20 and 21,
   wherein the component (10, 111, 69) is fixed to a plunger rod (11, 111) of the drug delivery device (1, 100) or wherein the component (10, 111, 69) of the drug delivery device (1, 100) is a housing (3) of the drug delivery device (1, 100).
23. The drug delivery device (1, 100) of any one of embodiments 20 to 22,
   wherein the distance between the distance sensor (39) and the component (10, 111, 69) of the drug delivery device (1, 100) is a distance between the distance sensor (39) and a surface (11a) of the component (10, 111, 69), in particular a surface (11a) at a distal end of the component (10, 111, 69) facing the distance sensor (39).
24. The drug delivery device (1, 100) of any one of embodiments 20 to 23,
   wherein at least a part of the surface of the component (10, 111, 69) is configured to reflect a probe signal (70) of the distance sensor (39) as a measurement signal (72) to be received by the distance sensor (39).
25. The drug delivery device (1, 100) of embodiment 24,
   wherein at least a part of the surface of the component (10, 111, 69) comprises a conductive and/or reflective section configured to produce the measurement signal (72), such as an electromagnetic echo, which can be received by the distance sensor (39) for determining the distance between the distance sensor (39) and the surface of the component (10, 111, 69),
26. The drug delivery device (1, 100) of embodiment 25,
   wherein the conductive and/or reflective section is a conductive and/or reflective section cover disposed on the surface (11a) of the component (10, 111, 69)
27. The drug delivery device (1, 100) of embodiment 25,
   wherein the conductive and/or reflective section is an integral part of the component (10, 111, 69).
28. The drug delivery device (1, 100) of any one of embodiments 20 to 27, wherein an interior space of the drug delivery device (1, 100) between the holder (80) and the component (10, 111, 69) is free from further components interacting with the probe signal (70) and/or the measurement signal (72) such that the probe signal (70) and/or the measurement signal (72) can travel unhindered between the holder (80) and the component (10, 111, 69).
29. The drug delivery device (1, 100) of any one of embodiments 20 to 28,
   wherein the drug delivery device (1, 100) comprises a sensing access (75) for sensing the position of the movable component (10, 111, 69) by distance sensor (68).
30. A set comprising an electronic module (27, 127) according to any one of embodiments 1 to 19 and a drug delivery device (1, 100) according to any one of embodiments 20 to 29.
31. A method of drug delivery, in particular to be executed by using the set according to the preceding embodiment, the method comprising
   generating a signal indicating a distance between a distance sensor (39) of an electronic module (27, 127) and at least one component (10, 111, 69) of a drug delivery device (1, 100) connected to the electronic module (27, 127),
   receiving the generated signal from the distance sensor (39), and acquiring, based on the received signal, information about at least one status of the drug delivery device (1, 100).
32. The method of the preceding embodiment,
   wherein the at least one status of the drug delivery device comprises one or several of a status indicating whether the electronic device (1, 100) is mounted on the drug delivery device (27, 127), a status indicating whether the drug delivery device (1, 100) is new or has already been used, a status indicating whether a dose setting has been performed on the drug delivery device (1, 100), a status indicating whether a dose delivery has started and a status indicating whether the dose delivery has been completed with the drug delivery device (1, 100).
33. The method at least one of embodiments 31 and 32,
   further comprising enabling dose dialing and/or dose dispensing with the drug delivery device (1, 100) by mounting the electronic module (27, 127) on the drug delivery device (1, 100).

Further embodiments of the present disclosure relate to the following embodiments:
1. An electronic module (27, 127) for a drug delivery device (1, 100),
   wherein the electronic module (27, 127) comprises a new pen sensor (37, 39, 90) configured to detect a position of at least one moveable, for example linearly moveable, component (10, 111, 69) of the drug delivery device (1, 100), and
   wherein the electronic module (27, 127), for example a processing unit (31) of the electronic module (27, 127), is configured to detect, based on a signal from the new pen sensor (37, 39, 90), whether the injection device (1, 100) is new or has already been used.
2. The electronic module (27, 127) of the preceding embodiment,
   wherein the new pen sensor (37, 39, 90) is different from a NFC circuit element and/or different from a frangible circuit.
3. The electronic module (27, 127) of at least one of the preceding embodiments,
   wherein the new pen sensor (37, 39, 90) is a new pen switch (37), for example with a flexible member, configured to generate the connection signal upon being actuated by the at least one moveable component (10, 111, 69) of the drug delivery device (1, 100).
4. The electronic module (27, 127) of embodiment 3,
   wherein the new pen sensor (37, 39, 90) is configured to be actuated by the at least one moveable component (10, 111, 69) via at least one intermediate component of the electronic module (27, 127) and/or the drug delivery device (1, 100).
5. The electronic module (27, 127) of at least one of the preceding embodiments,
   wherein the new pen sensor (37, 39, 90) is a distance sensor (39, 68) configured to measure a distance between the distance sensor (39, 68) and the at least one component (10, 111, 69) of the drug delivery device (1, 100) and configured to generate a signal indicative for the distance.
6. The electronic module (27, 127) of embodiment 5,
   wherein the electronic module (27, 127) is configured to compare the measured distance with a predetermined distance, which is, for example, stored in a memory of the electronic module (27, 127),
   wherein the electronic module (27, 127) is configured to detect that the injection device (1, 100) is new when the measured distance matches the predetermined distance or to detect that the injection device (1, 100) was already used when the measured distance does not match the predetermined distance.
7. A drug delivery device (1, 100) for an electronic module (27, 127) of at least one of the preceding embodiments,
   wherein the drug delivery device (1, 100) comprises a holder (80) for attachment of the electronic module (27, 127) and a moveable component (10, 111, 69),
   wherein the movable component (10, 111, 69) is configured to be positioned in an initial axial position with respect to the holder (80) when the drug delivery is new,
   wherein the movable component (10, 111, 69) is configured to leave the initial axial position upon a first use of the drug delivery device (1, 100),
   wherein the drug delivery device (1, 100) comprises a sensing access for sensing the position of the movable component (10, 111, 69) by the electronic module (27, 127).
8. The drug delivery device (1, 100) of embodiment 7,
   wherein the at least one movable component (10, 111, 69) is configured not to reach the initial position again after the first use of the drug delivery device (1, 100).
9. The drug delivery device (1, 100) of one of embodiments 7 and 8,
   wherein the at least one moveable component (10, 111, 69) of the drug delivery device (1, 100) is rigidly connected to a piston rod (11, 111) of the drug delivery device (1, 100), wherein, for example, the at least one moveable component (10, 111, 69) is a drive chassis (10) fixed to the piston rod (11) or is formed by the piston rod (111).
10. The drug delivery device (1, 100) of one of embodiments 7 and 8,
   wherein the at least one moveable component (10, 111, 69) of the drug delivery device (1, 100) is a protrusion which is disposed on an end section of the drug delivery device (1, 100) and which protrudes towards the electronic module (27, 127)
11. The drug delivery device (1, 100) of embodiment 10,
   wherein the protrusion is configured to mechanically deform a flexible member of the new pen sensor (37, 39, 90).

Further embodiments of the present disclosure relate to the following embodiments:
1. A drug delivery device (1, 100) comprising:
   a holder (80) for attachment of an electronic module (27, 127), and
   a lock (130) configured to prevent a dose setting operation and/or a dose delivery operation in a locked state and to allow the dose setting operation and/or the dose delivery operation in an unlocked state,
   wherein the lock (130) is in the locked state when the electronic module (27, 127) is not connected to the drug delivery device (1, 100) and wherein the lock (130) is configured to be in the unlocked state when the electronic module (27, 127) is connected to the drug delivery device (1, 100).
2. The drug delivery device (1, 100) of the preceding embodiment,
   further comprising a first component (10, 140) that is configured to perform a movement with respect to a second component (3) during the dose setting operation and/or the dose delivery operation,
   wherein the lock (130) is configured to lock the first component (10, 140) to the second component (3) in the locked state and to release the first component (10, 140) in the unlocked state.
3. The drug delivery device (1, 100) of embodiment 2,
   wherein the lock (130) comprises at least one locking member (132) configured to lock the first component (10, 140) to the second component (3) in the locked state,
   wherein the locking member (132) is configured to be at least partially displaceable by at least one unlocking component (134) when the electronic module (27, 127) is mounted on the drug delivery device (1, 100).
4. The drug delivery device (1, 100) of embodiment 3,
   wherein the locking member (132) is configured to directly engage with the unlocking component (134).
5. The drug delivery device (1, 100) of at least one of embodiments 3 and 4,
   wherein the unlocking component (134) is a part of the electronic module (27, 127).
6. The drug delivery device (1, 100) of at least embodiment 5,
   wherein the drug delivery device (1, 100) comprises an opening (135) to receive the unlocking component (134).
7. The drug delivery device (1, 100) of at least one of embodiments 3 and 4,
   wherein the unlocking component (134) is part of the drug delivery device (1, 100).
8. The drug delivery device (1, 100) of at least one of embodiments 2 to 7,
   wherein the drug delivery device (1, 100) comprises a locking element (23, 123) and a movable trigger (150),
   wherein the locking element (23, 123) locks the first component (10, 140) to the second component (3) prior to dose delivery,
   wherein the locking element (23, 123) is configured to be displaced by an unlocking movement of the trigger (150) to initiate dose delivery.
9. The drug delivery device (1, 100) of at least embodiment 8,
   wherein the locking member (132) prevents displacement of the locking element (23, 123) in the locked state,
   for example by engagement with the locking element (23, 123).
10. The drug delivery device (1, 100) of at least one of embodiments 8 and 9,
   wherein the locking member (132) prevents displacement of the locking element (23, 123) by restricting the movement of the trigger (150),
   for example by engagement with the trigger (150).
11. The drug delivery device (1, 100) of at least embodiment 10,
   wherein the lock (130) restricts, for example, blocks a movement of the trigger (150) in the locked state to a shorter distance than in the unlocked state,
   wherein, for example, the lock (130) restricts, for example, blocks the movement of the trigger (150) only in the locked state and not in the unlocked state.
12. The drug delivery device (1, 100) of at least one of embodiments 8 to 11,
   wherein the locking member (132) locks the first component (10, 140) to the second component (3) in addition to the locking element (23, 123) so that the first component (10, 140) and the second component (3) remain locked to each other in the locked state upon a displacement of the locking element (23, 123).
13. The drug delivery device (1, 100) of at least one of embodiments 3 to 12,
   wherein the locking member (132) comprises a flexible hinge and/or flexible protrusion connecting the first component (10, 140) with the second component (3).
14. The drug delivery device (1, 100) of at least one of embodiments 2 to 13,
   wherein the second component (3) comprises a housing (3) of the drug delivery device (1, 100).
15. The drug delivery device (1, 100) of at least one of embodiments 2 to 14,
   wherein the first component (10, 140) comprises a plunger rod (11, 111) of the drug delivery device (1, 100).
16. The drug delivery device (1, 100) of at least one of embodiments 2 to 15,
   wherein the first component (10, 140) comprises a drive sleeve (140) configured to drive the plunger rod (11, 111) during dose delivery.
17. The drug delivery device (1, 100) of at least one of embodiments 2 to 16,
   wherein the first component (10, 140) comprises a dose dial,
   wherein the dose dial is configured to be moved with respect to the second component (3) upon setting the dose to be delivered.
18. An electronic module (27, 127) for a drug delivery device (1, 100) according at least one of the preceding embodiments.
19. The electronic module (27, 127) of embodiment 18,
   wherein the electronic module (27, 127) comprises an unlocking component (134) for displacing a locking component (132) of the drug delivery device (1, 100),
   wherein, for example, the unlocking component (134) is a protrusion extending from the electronic module (27, 127).

Further embodiments of the present disclosure relate to the following embodiments:
1. An electronic module (27, 127) for a drug delivery device (1, 100),
   wherein the electronic module (27, 127) is configured to detect a setting of a dose and/or an end of a dose delivery process on a basis of binary switching events.
2. The electronic module (27, 127) of the preceding embodiment,
   wherein the electronic module (27, 127) comprises a dose setting sensor (93) configured to detect a first switching event of the binary switching events when the setting of the dose with the injection device (1, 100) has been completed.
3. The electronic module (27, 127) of at least embodiment 2,
   wherein the dose setting sensor (93) is configured to detect a binary switch status change upon completion of the setting of the dose.
4. The electronic module (27, 127) of at least embodiment 3,
   wherein the dose setting sensor (93) is configured to detect the binary switch status change only upon completion of the setting of the dose and at no further time during the dose setting and dose delivery process.
5. The electronic module (27, 127) of at least one of the preceding embodiments,
   wherein the electronic module (27, 127) comprises an end of dose sensor (95) configured to detect a second switching event of the binary switching events at the end of the dose delivery process, for example, upon completion of the dose delivery process.
6. The electronic module (27, 127) of at least embodiment 5,
   wherein the end of dose sensor (95) is configured to detect a binary switch status change at the end of the dose delivery process, such as upon completion of the dose delivery process.
7. The electronic module (27, 127) of at least embodiment 6,
   wherein the end of dose sensor (95) is configured to detect the binary switch status change only at the end of the dose delivery process, such as upon completion of the dose delivery process, and at no further time during the dose setting and dose delivery process.
8. The electronic module (27, 127) of at least one of embodiments 2 to 4 and at least one of embodiments 5 to 7,
   wherein the dose setting sensor (93) is separate from the end of dose sensor (95).
9. The electronic module (27, 127) of at least one of the preceding embodiments,
   wherein the electronic module (27, 127) is configured to store at least one of a date of the dose delivery process, a time of the dose delivery process, an amount of a set dose and an amount of a dose having actually been delivered.
10. A drug delivery device (1, 100) for an electronic module (27, 127) according to at least one of the preceding embodiments.
11. The drug delivery device (1, 100) according embodiment 10,
   wherein the drug delivery device (1, 100) is a fixed dose injection device allowing for delivery of a fixedly set dose.
12. The drug delivery device (1, 100) according to at least one of embodiments 10 and 11,
   wherein the drug delivery device (1, 100) is a multiple use injection device allowing repeated deliveries of the dose from a single medicament container attached to the drug delivery device (1, 100).

### Reference numeral list

- 1: drug delivery device
- 3: housing
- 3a: outer body
- 3b: inner body
- 5: cartridge/ syringe
- 7: plunger
- 9: driver
- 10: drive chassis
- 11: plunger rod
- 11a: reflective surface
- 12: optical pattern
- 13: drive spring
- 15: needle guard
- 16: needle guard spring
- 17: cap
- 19: needle shield
- 21: needle assembly
- 22: needle
- 23: locking element
- 25: resilient feature/acoustically active section
- 26: end of dose click protrusion
- 27: electronic module
- 28: PCBA
- 29: photo-reflector sensor
- 31: micro-controller
- 33: power supply
- 35: active wireless communicator
- 37: wake switch
- 39: time of flight sensor/ directional microphone
- 41: NFC connectivity system
- 43: frangible circuit element
- 45: RFID chip
- 47: antenna
- 49: attachment switch
- 51: sealing member
- 53: clip feature
- 55: protrusion
- 57: NFC tag
- 59: smartphone
- 61: barcode/ QR code
- 63: NFC reader
- 65: mechanical coding feature
- 67: window
- 68: distance sensor
- 69: movable component
- 70: probe signal
- 72: measurement signal
- 75: sensing access
- 80: holder
- 81: mechanical connector
- 82: electrical connector
- 85: communication unit
- 86: antenna
- 87: notifier
- 89: sensing electronics
- 90: new pen sensor
- 91: dispense sensor
- 92: sense circuit
- 93: dose setting sensor
- 94: sensing conductors
- 95: end of dose sensor
- 96: sensing conductors
- 97: identification sensor
- 98: designator
- 99: activation sensor
- 100: drug delivery device
- 101: needle end
- 103: housing ring
- 111: plunger rod
- 113: unidirectional coupling
- 115: drive lock
- 116: first sensor
- 118: second sensor
- 120: circuit lines
- 122: contact
- 123: locking element
- 124: flexible contact
- 125: circuit line
- 127: electronic module
- 128: intermediate component
- 129: flexible connection
- 130: lock
- 132: locking member
- 134: unlocking component
- 135: opening
- 140: drive sleeve
- 141: proximal part
- 142: distal part
- 150: trigger
- 160: dose dial

## Claims

1. An electronic module (27, 127) for a drug delivery device (1, 100), wherein the electronic module (27, 127) comprises
a distance sensor (68) configured to generate a signal indicating a distance between the distance sensor (68) and at least one component (10, 111) of the drug delivery device (1, 100) connected to the electronic module (27, 127), and
a processing unit configured to receive the generated signal from the distance sensor (68) and to acquire, based on the received signal, information about at least one status of the drug delivery device (1, 100).

2. The electronic (27, 127) module according to claim 1,
wherein the distance sensor (68) is different from a time-of-flight sensor.

3. The electronic module (27, 127) according to claim 1 or 2,
wherein the distance sensor (68) is based on a phase-shift measurement, a triangulation measurement and/or an intensity modulation measurement.

4. The electronic module (27, 127) according to at least one of the preceding claims,
wherein the distance sensor (68) is an ultrasonic sensor, a RADAR sensor, a LIDAR sensor and/or an IR sensor.

5. The electronic module (27, 127) according to at least one of the preceding claims,
wherein the distance sensor (68) is positioned on a proximal side of the electronic module (27, 127) facing the attached drug delivery device (1, 100).

6. The electronic module (27, 127) according to at least one of the preceding claims,
wherein the distance sensor (68) is a contactless distance sensor configured to emit a probe signal (70) towards the component (10, 111) of the drug delivery device (1, 100) and to receive a reflection of the probe signal as a measurement signal (72) from the drug delivery device (1, 100).

7. The electronic module (27, 127) according to claim 6,
wherein the distance sensor (68) is configured to emit the probe signal (70) and/or to receive the measurement signal (72) in a direction essentially parallel, such as parallel, to a longitudinal axis of the drug delivery device (1, 100).

8. The electronic module (27, 127) according to at least one of the preceding claims,
wherein the information about the at least one status of the drug delivery device (1, 100) comprises at least one of information about whether the drug delivery device (1, 100) is new or has already been used, information about whether a mounting of the electronic module (27, 127) on the drug delivery device (1, 100) has been successful, a date of the successful mounting, a time of the successful mounting, a number of mounting attempts, an amount of a dose having been set during a dose dialing, a date of the dose dialing, a time of the dose dialing, information about whether a dose has been dispensed, such as completely dispensed, during a dose dispensing, an amount of a dose having been dispensed during the dose dispensing, a date of the dose dispensing and a time of the dose dispensing.

9. A drug delivery device (1, 100) comprising a holder (80) for holding the electronic module (27, 127) according to at least one of claims 1 to 8,
wherein, for example, the electronic module (27, 127) is attachable to and detachable from the drug delivery device (1, 100) or is permanently fixed to the drug delivery device (1, 100).

10. The drug delivery device (1, 100) according to claim 9,
wherein the component (10, 111) of the drug delivery device (1, 100) and the holder (80) are configured to perform at least a linear movement relative to each other upon the dose dialing and/or dose dispensing.

11. The drug delivery (1, 100) device according to at least one of claims 9 and 10,
wherein the component (10, 111) comprises a plunger rod (11, 111) of the drug delivery device (1, 100) or
wherein the component of the drug delivery device (1, 100) is a part of a housing (3) of the drug delivery device (1, 100).

12. The drug delivery device (1, 100) according to at least one of claims 9 to 11,
wherein the distance between the distance sensor (68) and the component (10, 111) of the drug delivery device (1, 100) is a distance between the distance sensor (68) and a surface (11a, 112) of the component (10, 111), in particular a surface (11a, 112) at a distal end of the component (10, 111) facing the distance sensor (68).

13. The drug delivery device (1, 100) according to at least claim 12,
wherein at least a part of the surface (11a, 112) of the component (10, 111) is configured to reflect a probe signal (70) of the distance sensor (68) as a measurement signal (72) to be received by the distance sensor (68),
wherein, for example, at least a part of the surface (11a, 112) of the component (10, 111) comprises a conductive and/or reflective section configured to produce the measurement signal (72), such as an electromagnetic echo, which can be received by the distance sensor (68) for determining the distance between the distance sensor (68) and the surface (11a, 112) of the component (10,111), the conductive and/or reflective section being a conductive and/or reflective section cover disposed on the surface of the component or the conductive and/or reflective section being an integral part of the component.

14. The drug delivery device (1, 100) according to claim 13,
wherein an interior of the drug delivery device (1, 100) between the holder (80) and the component (10, 111) is free from components interacting with the probe signal (70) and/or the measurement signal (72) such that the probe signal (70) and/or the measurement signal (72) can travel unhindered between the holder (80) and the component (10,111).

15. A set comprising the electronic module (27, 127) according to at least one of claims 1 to 8 and the drug delivery device (1, 100) according to at least one of claims 9 to 14.

16. A method of drug delivery, in particular to be executed by using the set according to claim 15, the method comprising:
generating a signal indicating a distance between a distance sensor (68) of an electronic module (27, 127) and at least one component (10, 111) of a drug delivery device (1, 100) connected to the electronic module (27, 127),
receiving the generated signal from the distance sensor (68), and
acquiring at least one of information about a process of mounting the electronic device on the drug delivery device (1, 100), information about a dose setting performed on the drug delivery device (1, 100) and information about a dose dispensing performed on the drug delivery device (1, 100).

17. The method according to claim 16, further comprising:
before the generating of the signal, determining whether the drug delivery device (1, 100) is new or has already been used.

18. The method according to claim 16 or 17, further comprising:
before the determining whether the drug delivery device (1, 100) is new or has already been used, enabling the dose dialing and/or dose dispensing with the drug delivery device (1, 100) by mounting the electronic component (27, 127) on the drug delivery device (1, 100).
